# EUROPEAN PATENT SPECIFICATION

(11) **EP 4 367 226 B1**
(45) Date of publication and mention of the grant of the patent: **11.02.2026**
(21) Application number: 22744165.6
(22) Date of filing: 04.07.2022
(51) Int. Cl.: C12N 9/12, C07K 16/40, C07K 14/52, C07K 14/715, C30B 29/58, G16B 15/30

(54) **CRYSTAL STRUCTURES OF ALK AND LTK RECEPTOR TYROSINE KINASES AND THEIR LIGANDS**
KRISTALLSTRUKTUREN VON ALK- UND LTK-REZEPTORTYROSINKINASEN UND DEREN LIGANDEN
STRUCTURES CRISTALLINES DE TYROSINE KINASES DE RÉCEPTEUR ALK ET LTK ET LEURS LIGANDS

(30) Priority: 07.07.2021 EP 21184332
(43) Date of publication of application: 15.05.2024
(73) Proprietor: VIB VZW, 9052 Gent (BE); Universiteit Gent, 9000 Gent (BE)
(72) Inventor: SAVVIDES, Savvas, 8500 Kortrijk (BE); DE MUNCK, Steven, 9120 Melsele (BE)
(86) International application number: PCT/EP2022/068425
(87) International publication number: WO 2023/280766

(56) References cited:
- WO-A1-2014/159074
- JIKUI GUAN ET AL: "FAM150A and FAM150B are activating ligands for anaplastic lymphoma kinase", ELIFE, 29 September 2015 (2015-09-29), XP055764931

## Description

### Field of the invention

The present invention belongs to the field of structural biology, more particularly to the field of cytokine biology. In particular the invention provides co-crystals of the anaplastic lymphoma kinase (ALK) and its ligand ALKAL2 and the related leukocyte tyrosine kinase (LTK) and its ligand ALKAL1. The invention also provides computer-assisted and other methods for selecting molecules able to modulate the interaction between ALK, LTK with their respective ligands.

### Introduction to the invention

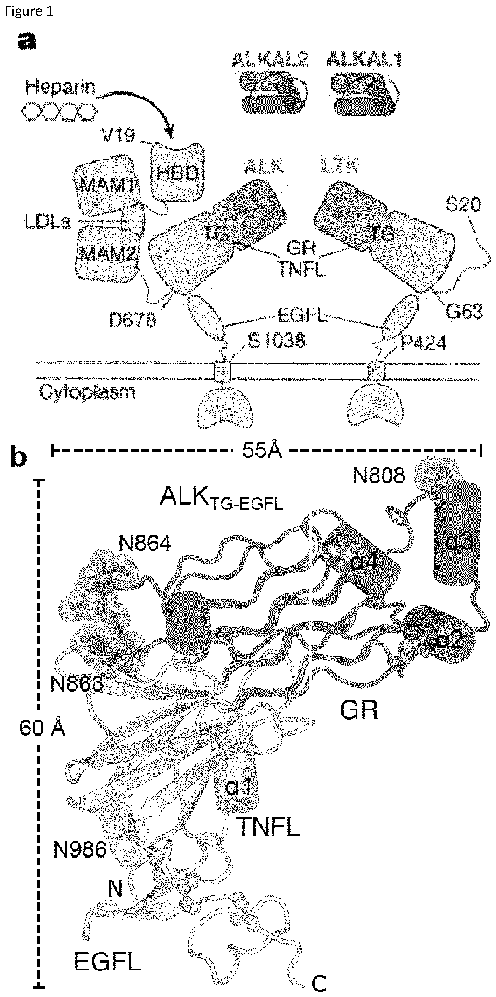

The architectural hallmark of ALK family receptors is a membrane-proximal segment in their extracellular domain marked by multiple stretches of glycine residues coupled to an EGF-like (EGFL) module. The glycine-rich composition of this segment complicates detection of a globular fold motif but has led to its sequence-based classification as Glycine-rich PFAM domain PF12810⁴. Faint, local similarity of a predicted beta-strand segment in ALK with part of the TNF superfamily beta-jellyroll scaffold led to a proposed delineation of a TNF-like (TNFL) module preceding the glycine-rich (GR) region of ALK and LTK⁵. Whereas this module together with EGFL constitutes the bulk of LTK, ALK builds a much more substantial extracellular segment comprising an MAM-LDLa-MAM domain cassette and an N-terminal heparin binding domain (HBD) (Fig. 1a). Activation of ALK family receptors requires binding of two recently discovered cytokines ALKAL1 and ALKAL2 (also called FAM150A/FAM150B, and AUGbeta/AUGalfa)⁶⁻⁸ to the membrane-proximal modules common to LTK and ALK. ALKAL1 and ALKAL2 share high sequence identity (66%) in their C-terminal domain but have variable N-terminal regions. Whereas both cytokines are strong activators of LTK⁷, only ALKAL2 potently induces ALK signalling^{5,8}, which is additionally regulated via glycosaminoglycan-mediated receptor dimerization through ALK's N-terminal HBD⁹.

Remaining functionally enigmatic, ALK is best known for its involvement in cancer¹⁰, such as non-small cell lung cancer and pediatric neuroblastoma⁸. Moreover, ALKAL1 has been linked to BRAF inhibitor resistance in melanoma¹¹ and ALKAL2 to poor survival in neuroblastoma patients¹². The disease context of LTK is less clear and currently situates in autoimmune disorder lupus¹³ and acute myeloid leukemia¹⁴. Interestingly, the newly proposed role of ALK in metabolic control also appears to be present in Drosophila indicating an evolutionary conservation of ALK function¹⁵. As ALK family receptors and cognate cytokines are gaining therapeutic importance^{16,17} the field is limited by the stark paucity of structural and mechanistic insights, in contrast to most other RTK.

The present invention satisfies this need and provides 3D-structural models of ALK and LTK respectively binding to their ligands ALKAL2 and ALKAL1.

### Figure legends

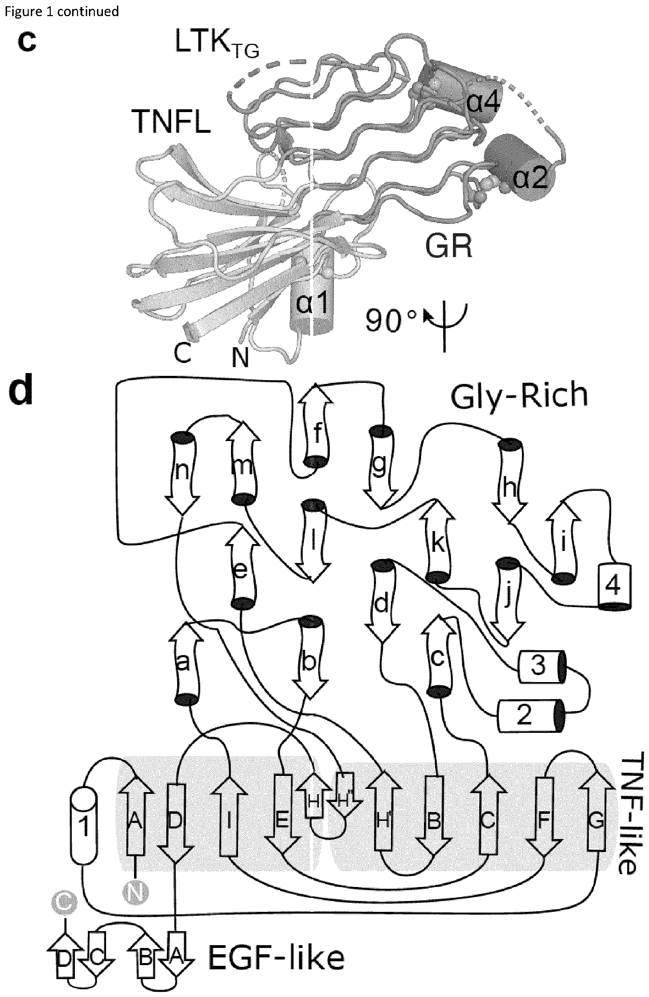

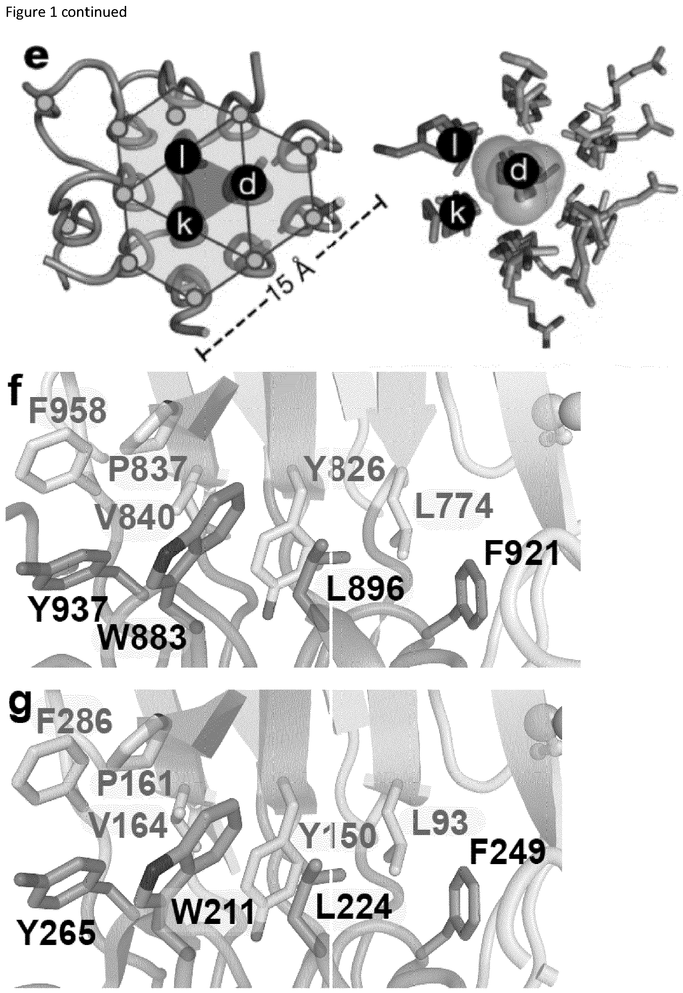

Figure 1. Structure of a novel cytokine-binding domain in ALK and LTK.
   a, Schematic representation of ALK family receptors ALK and LTK with binding partners participating in signalling complexes. Abbreviations are EC, extracellular; PM, plasma membrane; IC, intracellular; HBD, heparin-binding domain; MAM, meprin/A5-protein/PTPµ domain; TG, TNF-like/Glycine-rich supradomain; EGF-like, epidermal growth factor-like domain; LDLa, Low-density lipoprotein class A repeat.
   b,c Cartoon representation of crystal structures of ALK_{TG-EGFL}(b) and LTK_{TG} (c), with TNFL is pink (ALK) and light-yellow (LTK), and the GR in purple (ALK) and orange (LTK). Disulfides are shown as yellow spheres. The boxed area denotes the hydrophobic groove between the TNFL and GR subdomains. The four experimentally observed N-linked glycans in ALK at positions N808, N863, N864 within the TG supradomain, and N986 in the linker adjoining the TG and the EGF-like domain are depicted as transparent spheres embedded with stick representations of the modelled glycans.
   d, Topology diagram of the TG supradomain. β-strands (green), α-helices (blue), pGll helices (orange) and the three central pGll helices d,k and I (vermillion). Different β-sheets are denoted with a light blue background.
   e, (left) Representations of the pGII-helix arrangement of ALK with the central pGll-helices d,k and I highlighted in the central triangle. (right) A subset of the LTK pGll-helices are shown in stick representation with central pGll-helix d in spheres.
   f,g Close-up view of the hydrophobic interface between the H'HGFCB sheet in the TNFL subdomain and the glycine-rich subdomain of ALK (f) and LTK (g).

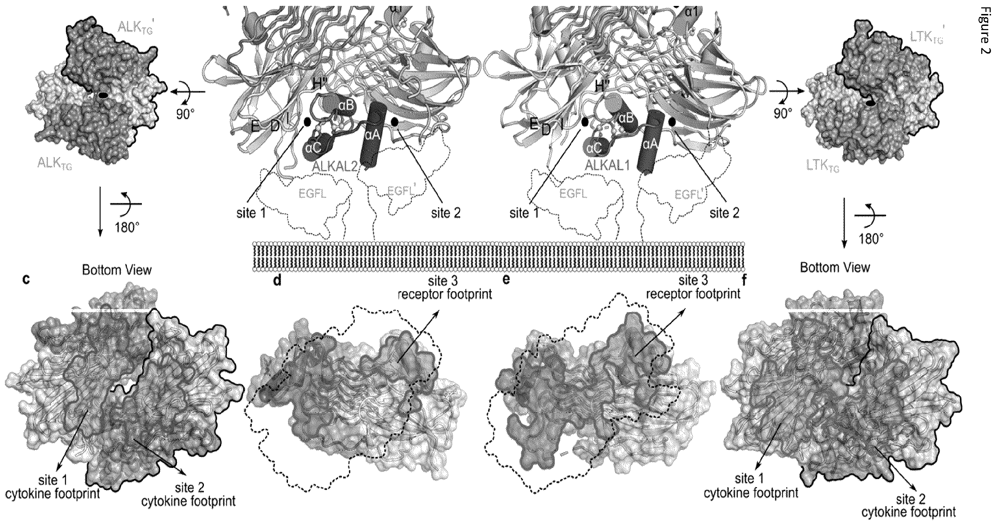

Figure 2. Structure of the ALK_{TG}-ALKAL2 and LTK_{TG}-ALKAL1 ternary complexes.
   a,b Crystal structures of the ternary assembly of ALK_{TG} (pink, gray) with ALKAL2 (blue) (a) and the ternary assembly of LTK_{TG} (orange, gray) with ALKAL1 (green) (b). The helices of both ALKAL1 and 2 are labeled αA, αB and αC. Helices of ALK and LTK are labeled α1, α2, α3 and α4. The β-strands of the cytokine binding epitope in ALK and LTK are labeled E, D, H" and I. The position of the EGF-like domains after alignment of ALK_{TG-EGFL} with ALK and LTK in the complex is indicated by a surface outline. Top view of the ternary complex, the black ellipse indicates the two-fold symmetry axis.
   c, Bottom view of the ALK_{TG}-ALKAL2 complex the total buried interaction interfaces of site 1 (755 Å²) and 2 (630 Å²) with ALKAL2 are indicated with a blue overlay.
   d, Front view of the ALK_{TG}-ALKAL2 complex the total site 3 (760 Å²) buried interaction interface between the two receptor copies is indicated with a red overlay. One ALK copy is represented by a dotted surface outline.
   e, Front view of the LTK_{TG}-ALKAL1 complex the total site 3 (880 Å²) buried interaction interfaces with ALKAL1 between the two receptor copies is indicated with a red overlay. One LTK copy is represented by a dotted surface outline.
   f, Bottom view of the LTK_{TG}-ALKAL1 complex the total buried interaction interface of site 1 (740 Å²) and 2 (580 Å²) are indicated with a green overlay.

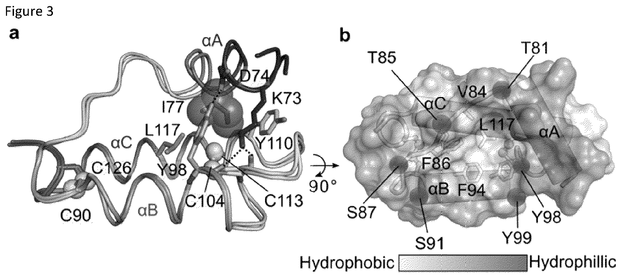

Figure 3. ALKAL1 and ALKAL2 adopt an open three-helix bundle fold.
   a, Overlay of ALKAL1 and ALKAL2 coloured in an N- (blue) to C-terminus (red) gradient. ALKAL1 residues in the interface between helix A and helices B and C are shown as sticks.
   b, Surface view of ALKAL1 coloured according to the Eisenberg hydrophobicity scale. Hydroxyl groups surrounding the central cavity are shown as red spheres.

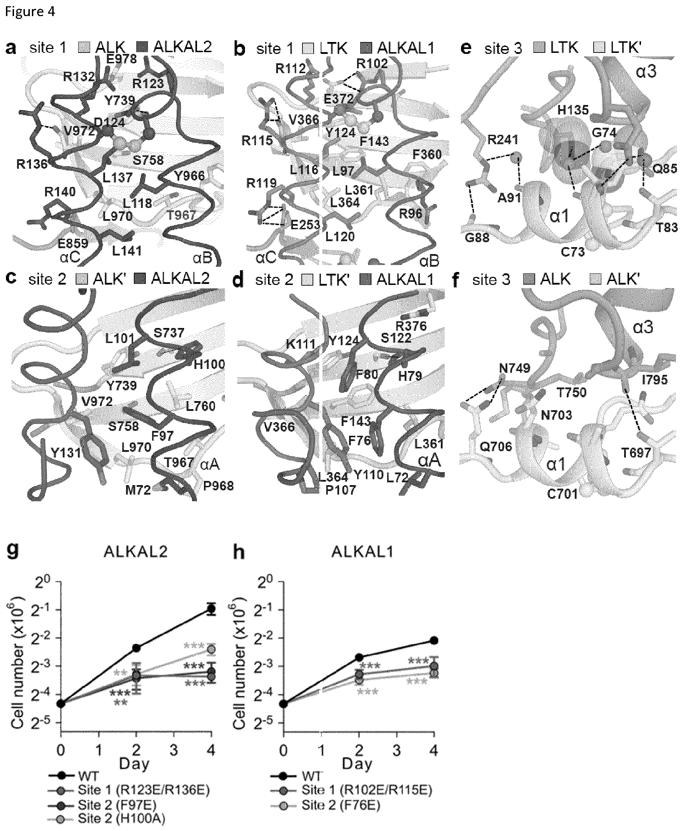

Figure 4. ALK/LTK-cytokine complexes display 3 distinct interaction sites.
   a,b Close-up view of site 1 in the ALK-ALKAL2 (a) and LTK-ALKAL1 (b) complexes. ALKAL1 residues investigated by mutagenesis are coloured red.
   c,d Close-up view of site 2 in the ALK-ALKAL2 (c) and LTK-ALKAL1 (d) complexes. ALKAL2 residues investigated by mutagenesis are coloured red.
   e,f Close-up view of site 3 in the LTK-ALKAL1 (e) and ALK-ALKAL2 (f) complexes.
   g,h Cell proliferation of Ba/F3 cells expressing ALK^{WT} with ALKAL2^{WT} and the R123E/R136E, F97E and H100A mutants (g) or with ALKAL1^{WT} and the R102E/R115E and F76E mutants (h). (*n* = 3 biologically independent experiments; mean ± s.d.; two-way ANOVA with Tukey's multiple comparison test compared with WT. *P < 0.05, ***P <* 0.01 and ***P < 0.001).

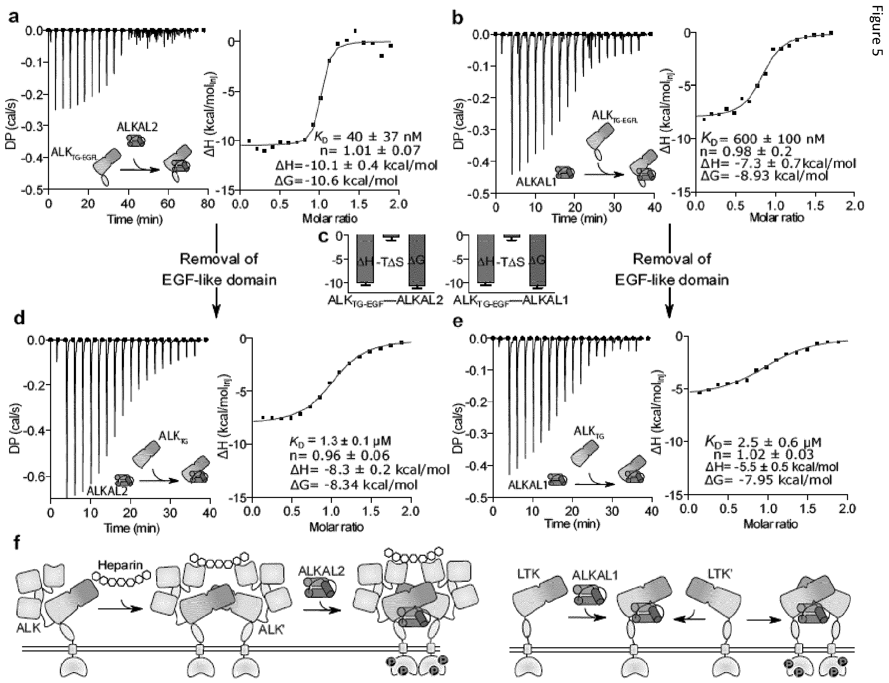

Figure 5. The EGF-like domain of ALK mediates cytokine specificity.
   a-b Isothermal titration calorimetry thermograms for the titration of ALK_{TG-EGFL}(10 µM) with ALKAL2 (100 µM) (a) and titration of ALKAL1 (25 µM) with ALK_{TG-EGFL} (210 µM) (b). For each titration the left panel shows the raw data with the differential power (DP) plotted against time. The right panel represents the binding isotherm obtained from the integration of the raw data and fitted to a one-site model. Standard deviations were calculated based on 3 measurements.
   c, Thermodynamic footprint of the ALK_{TG-EGFL}-ALKAL2 and ALK_{TG-EGFL}-ALKAL1 interaction d, Titration of ALKAL2 (33 µM) with ALK_{TG} (330 µM) Standard deviations were calculated based on 3 measurements.
   e, Titration of ALKAL1 (33 µM) with ALK_{TG} (330 µM) Standard deviations were calculated based on 3 measurements.
   f, Assembly mechanism of ALK family complexes. ALKAL mediated ALK signaling may require additional dimerization by heparin while LTK activation is completely cytokine driven.

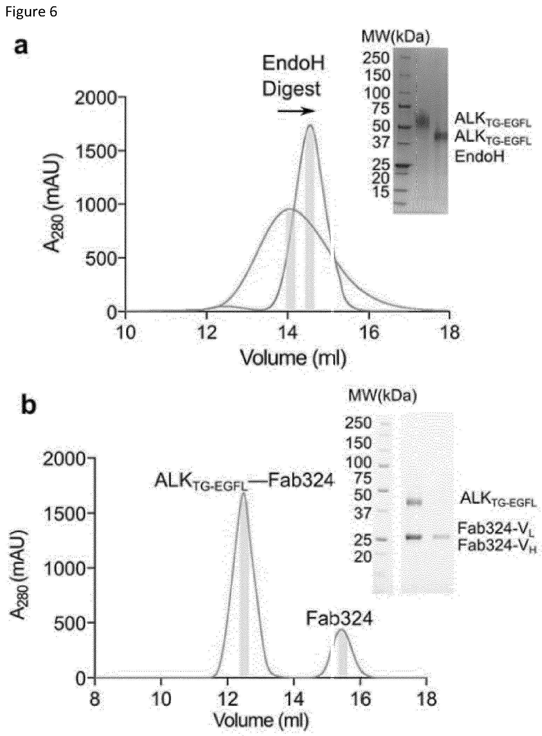

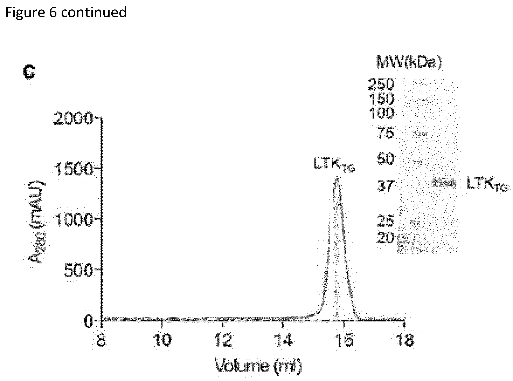

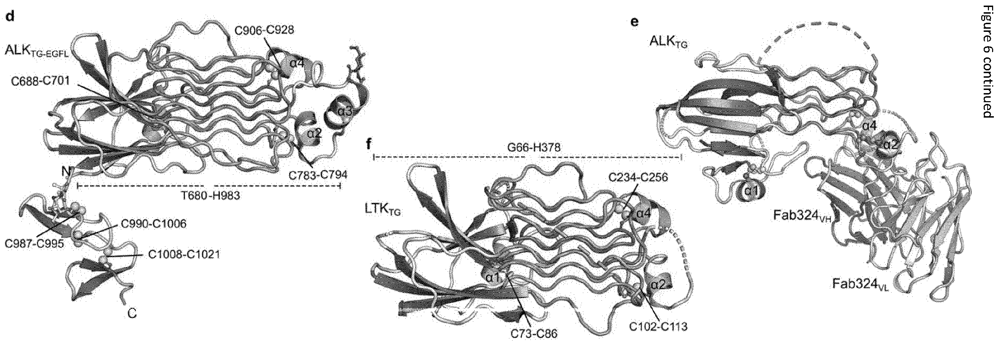

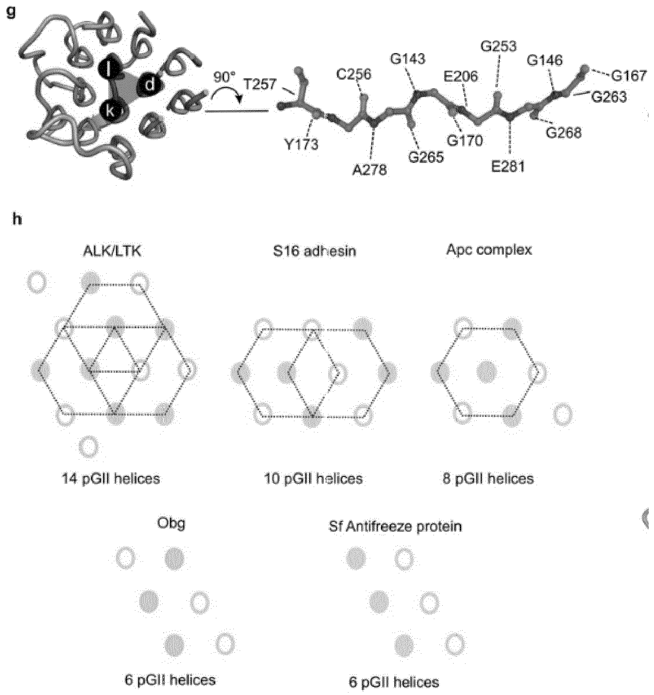

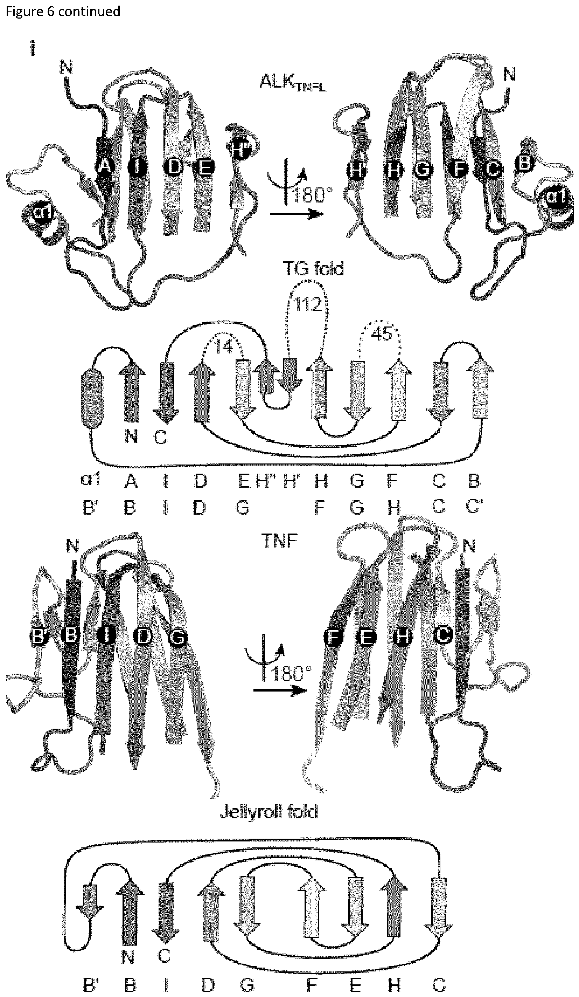

Figure 6. Purification of ALK, LTK and structural details of the TG supradomain fold.
   a,b,c Representative chromatograms and SDS-PAGE gels for the purification of ALK_{TG-EGFL} (a), ALK_{TG-EGFL}-Fab324 (b) and LTK_{TG} (c). The arrow indicates the shift in elution volume after EndoH digest of ALK_{TG-EGFL}. d, ALK_{TG-EGFL} structure colored according to secondary structure elements. α-Helices (blue), β-strands (green), pGll-helices (orange), loops (grey).
   e, Structure of the ALK_{TG-EGFL}-Fab324 complex with ALK coloured according to its secondary structure elements. CDR loops of Fab324 are coloured yellow. The constant domains of Fab324 are omitted for clarity.
   f, LTK_{TG} structure colored according to secondary structure elements.
   g, Hexagonal pGll helix arrangement surrounding pGll helix d in LTK. Vermillion pGll-helices consist exclusively of glycine residues. pGll helix d shown as sticks, hydrogen bonds to other residues in LTK are indicated as dotted lines.
   h, Schematic representations of available pGll helical arrangements. Full circles indicate pGll helices coming out of the plane of the page while empty circles indicate helices going into the plane of the page. S16 adhesin (pdb: 6F45) Apc complex (pdb: 5L9W) obg (pdb: 5M04) Sf antifreeze protein (pdb: 3BOI)
   i, The β-sheet containing subregions of ALK and a trimmed view of TNF (pdb: 1TNF) are coloured in a N-(blue) to C-terminus (red) gradient and shown side by side after structural superposition. Topology diagram for the TNFL domain of ALK and the jelly-roll fold of TNF follow the same colour scheme. Jelly-roll fold nomenclature starts with strand B according to convention. For ALK_{TNFL} the nomenclature in black is according to the TG domain notation used in this study while the nomenclature according to the TNF convention (first β-strand labeled B) is shown in grey. The sequential B to I β-strands of the TNF/C1q β-jellyroll smoothly sew together the two β-sheets (that feature characteristic B'BIDG and FEHC faces) whereas the ALK/LTK_{TNFL} subdomain has AIDEH" and H'HGFCB faces (primed small caps denote additional, edge β-strands).

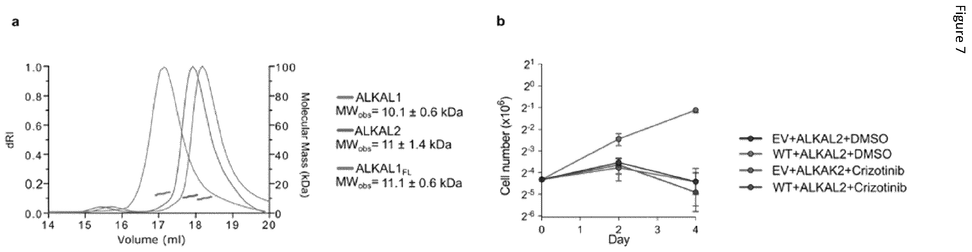

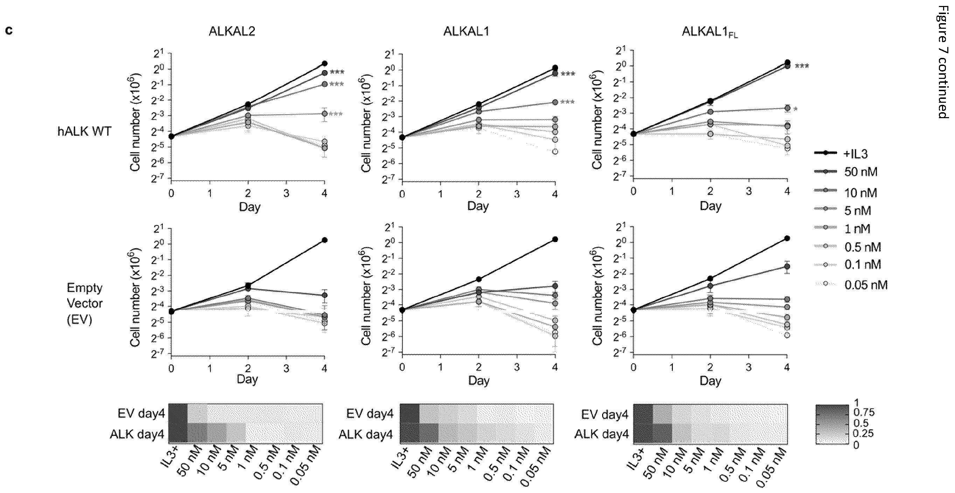

Figure 7. Activity and biophysical characterization of ALKAL1 and ALKAL2.
   a, Molecular mass determination of ALKAL2, ALKAL1 and ALKAL1_{FL} by size-exclusion chromatography and in-line multi-angle laser light scattering (SEC-MALLS). Differential refractive index (left axis) is plotted against the determined molecular weight (right axis). ALKAL2 (blue trace), ALKAL1 (green trace) and ALKAL1_{FL} (vermillion trace).
   b, Cell proliferation of ALK^{WT} or EV (empty vector) expressing Ba/F3 cells treated with 10mM ALKAL2 with and without addition of crizotinib. (*n* = 3 biologically independent experiments; mean ± s.d)
   c, Cell proliferation of Ba/F3 cells expressing ALK^{WT} or EV upon stimulation by a concentration series of ALKAL2, ALKAL1 or ALKAL1_{FL} at indicated concentration. Standard deviations are calculated from 3 technical replicates. The ratio of the observed ALKAL-induced cell growth with the IL-3 induced cell growth is shown in a heatmap representation for the measurements on day 4. **P* < 0.05, ***P* < 0.01 and ****P* < 0.001.

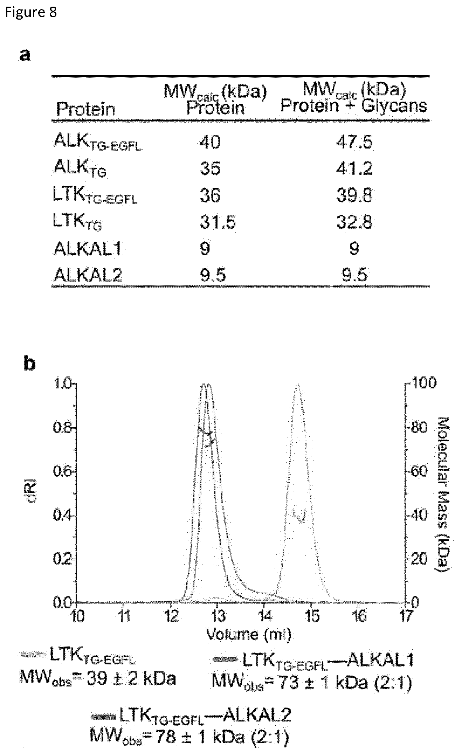

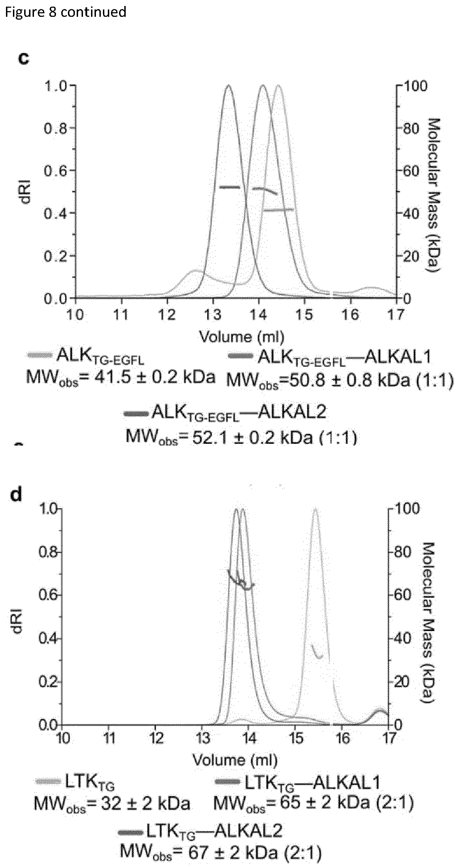

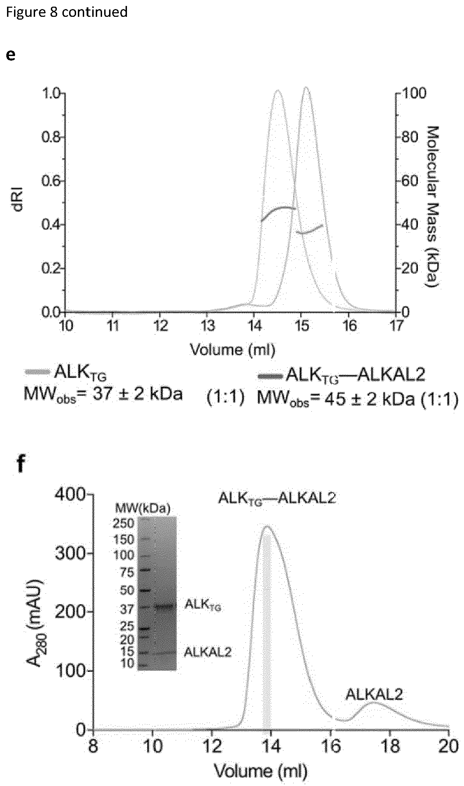

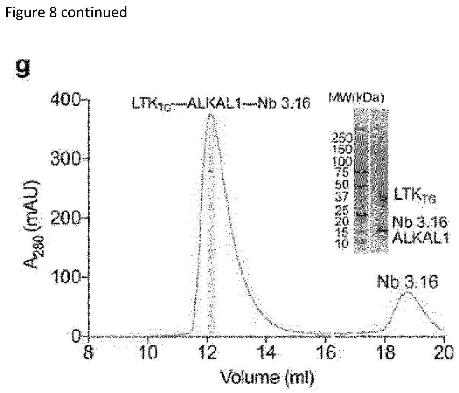

Figure 8. Biophysical characterization and purification of ALKAL mediated complexes with ALK and LTK. a, Calculated theoretical molecular weights for the ALK, LTK and ALKAL constructs under study.
   b-e, Molecular mass determination of ALKAL mediated complexes with LTK_{TG-EGFL} (b), ALK_{TG-EGFL} (deglycosylated) (c), LTK_{TG} (d), ALK_{TG} (deglycosylated) (e)Differential refractive index (left axis) is plotted against the determined molecular weight (right axis). Uncomplexed LTK and ALK constructs are in orange and pink respectively. Complexes with ALKAL1 or ALKAL2 are in green and blue respectively.
   f,g Representative chromatograms and SDS-PAGE gels for the purification of the ALK_{TG-EGFL}-ALKAL2 complex (f) and the LTK_{TG-EGFL}-ALKAL1-Nb3.16 complex (g).

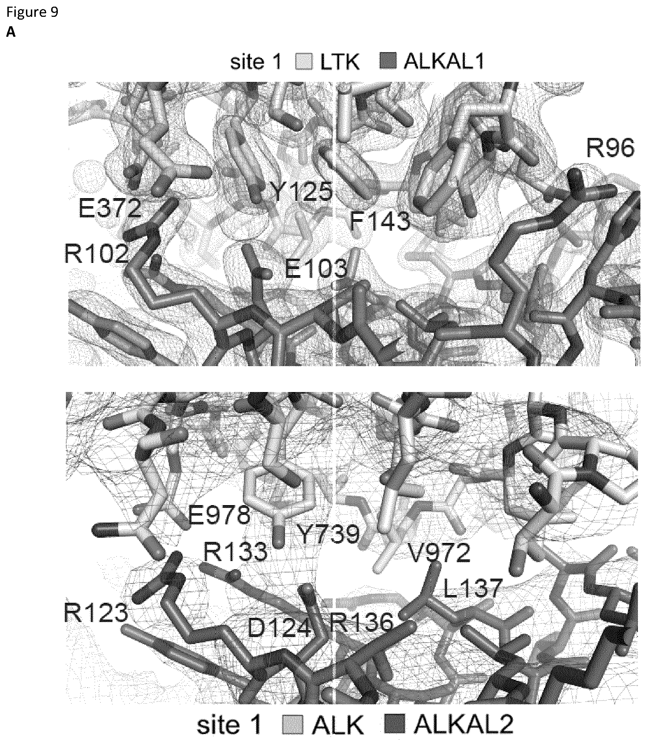

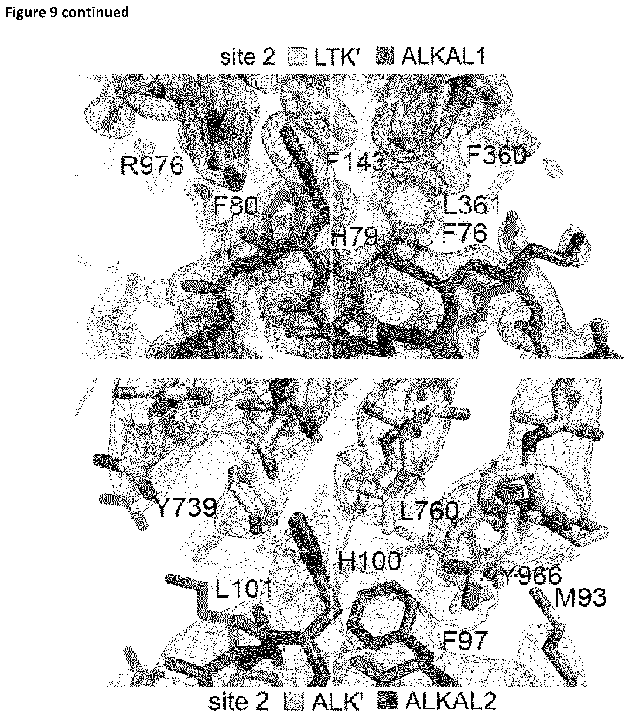

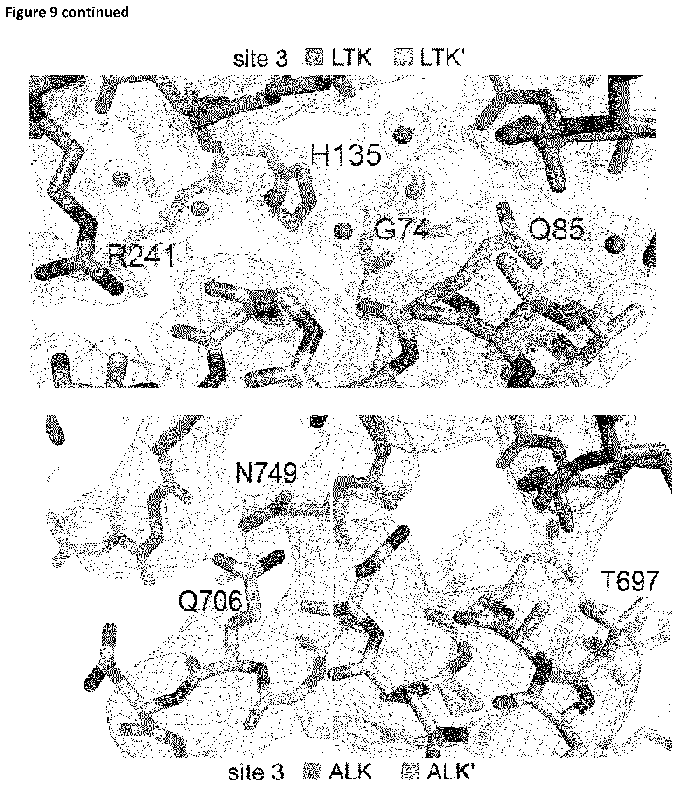

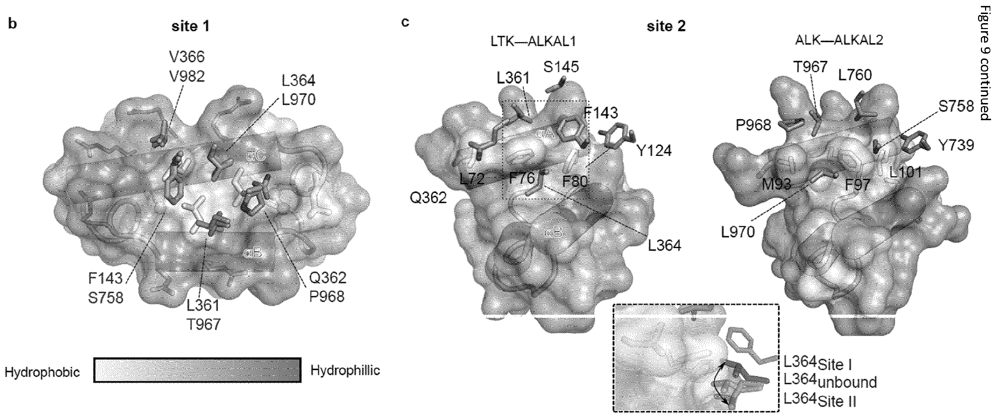

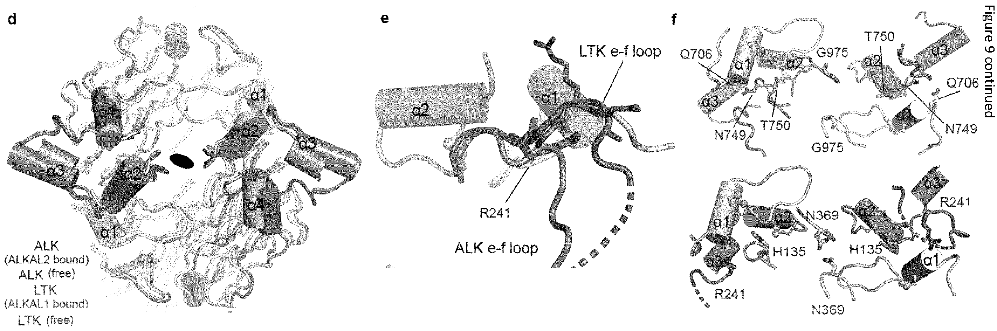

Figure 9. Structural details of receptor-cytokine and receptor-receptor interactions in ALK/LTK-cytokine complexes.
   a, 2Fo-Fc electron density maps contoured at +1 r.m.s.d. showing details of site 1, 2 and 3 of the LTK-ALKAL1 and ALK-ALKAL2 complexes.
   b, ALKAL1 shown in transparent surface coloured according to the Eisenberg hydrophobicity scale. Showing the central conserved hydrophobic patch formed by leucines (L97, L116 and L120) with the interacting residues of LTK (orange). The equivalent ALK residues (pink) are shown after alignment with LTK.
   c, View of Site 2 in both the LTK-ALKAL1 and ALK-ALKAL2 complexes. ALKALs are coloured according to the Eisenberg hydrophobicity scale. Receptor residues surrounding the hydrophobic triad of helix A (L72, F76, F80 in ALKAL1 and M93, F97, L101 in ALKAL2) are shown as sticks for LTK (orange) and ALK (pink).
   d, Superposition of free ALK (dark gray), free LTK (light gray), bound ALK (pink, only helices shown) and bound LTK (orange, only helices shown).
   e, Superposition of the ALK-ALKAL2 and LTK-ALKAL1 complexes, zoomed in on the region around the e-f loop.
   f, View of the site 3 groove of ALK (top) and LTK (bottom).

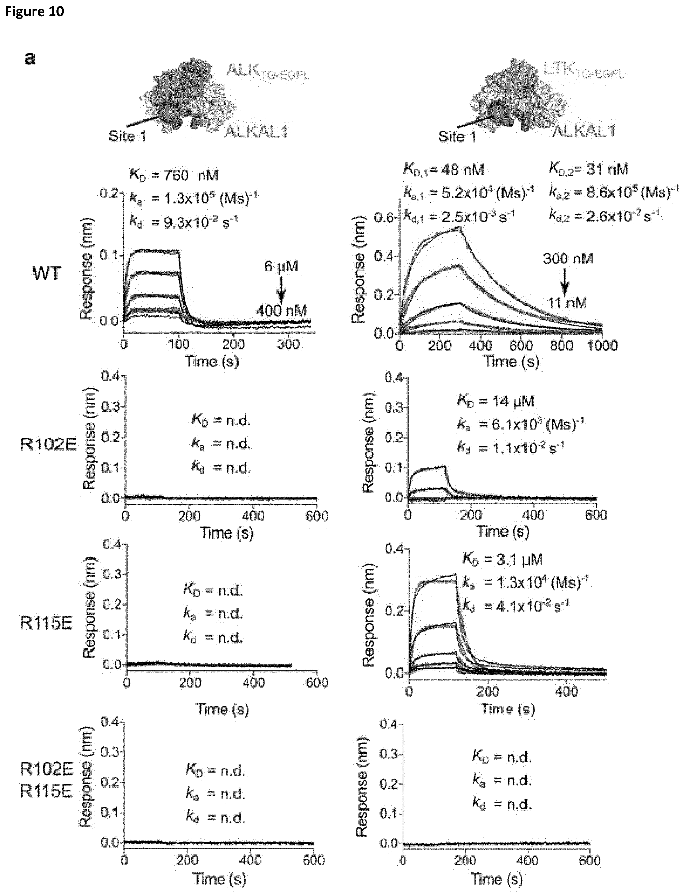

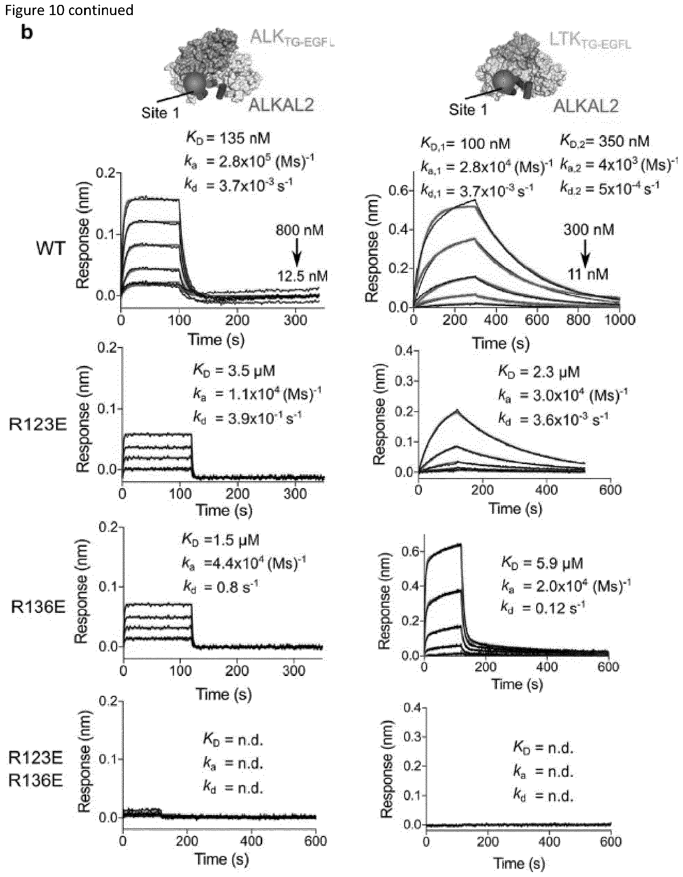

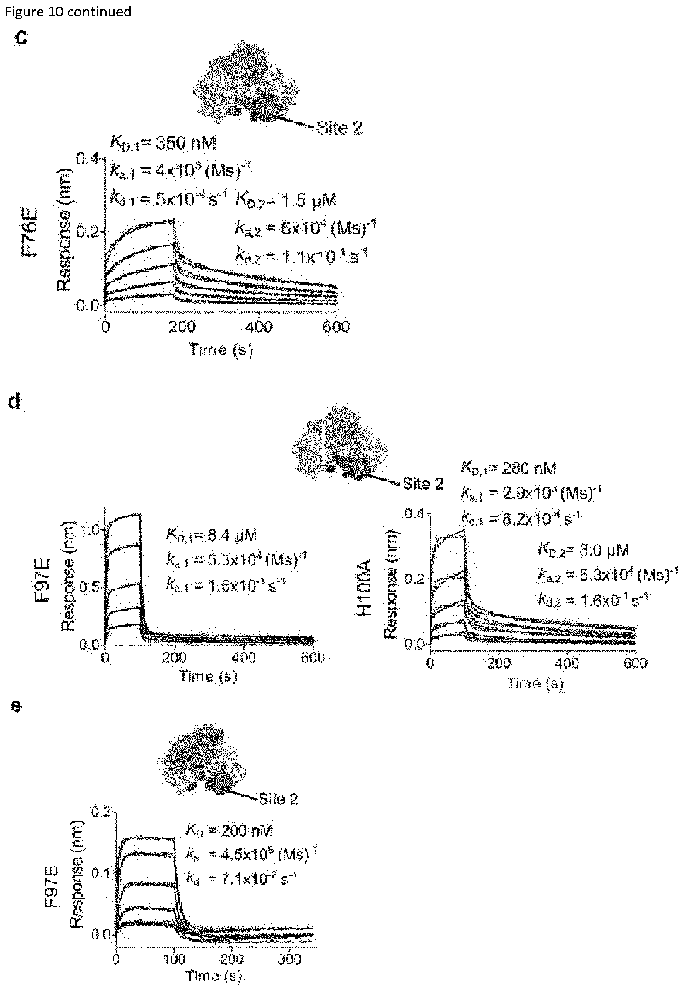

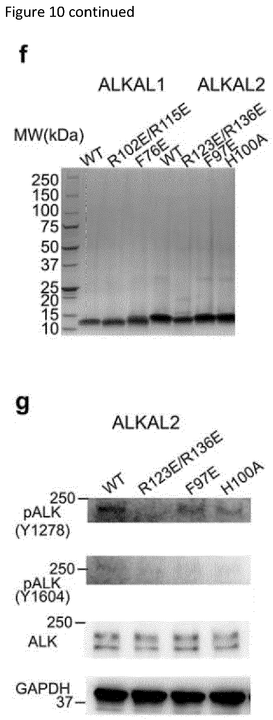

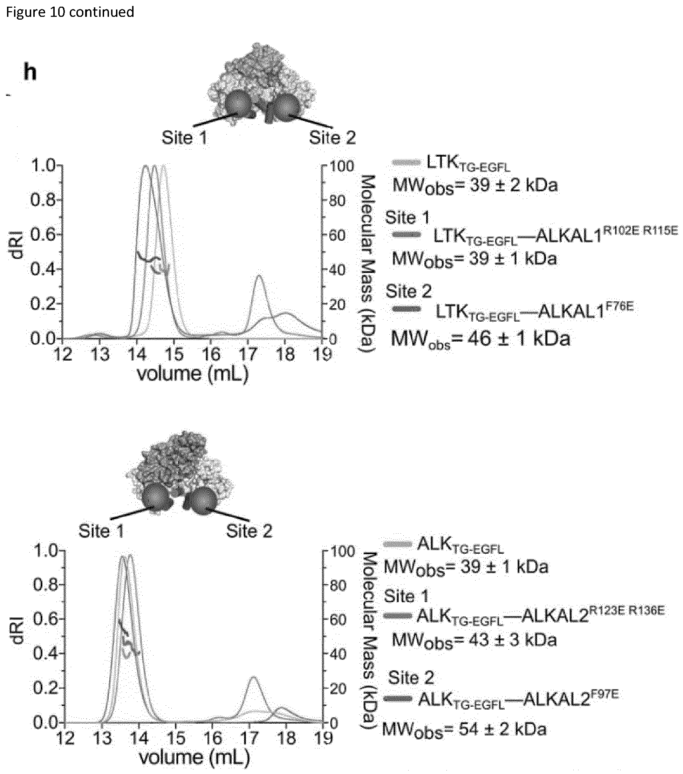

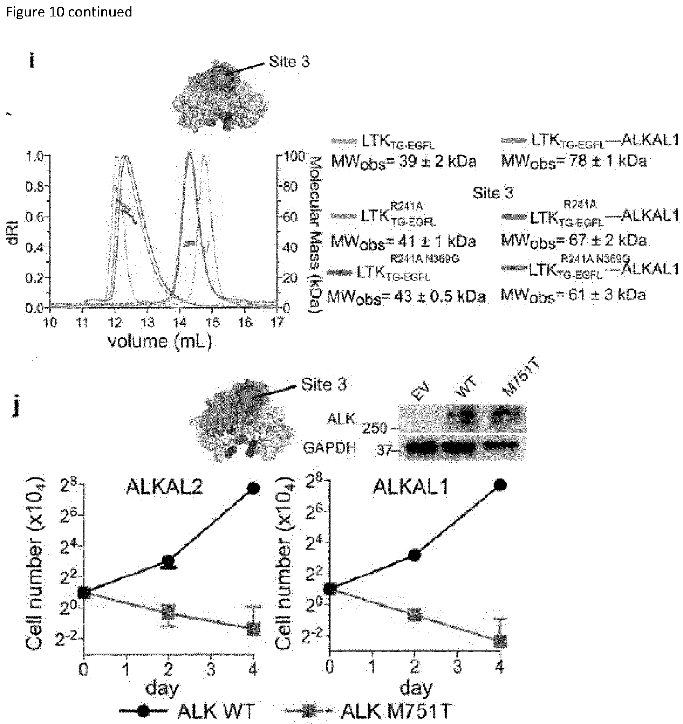

Figure 10. Functional interrogation of site 1, site 2, and site 3 interfaces in ALK/LTK-cytokine complexes. a,b Representative response curves as measure by biolayer-interferometry (BLI) for the interaction of WT ALKAL1 and ALKAL1 mutants (containing charge-reversal mutations of residues involved in site 1) (a) and WT ALKAL2 and ALKAL2 mutants (b) with ALK_{TG-EGFL} and LTK _{TG-EGFL}. For WT ALKALs LTK curves were fitted with a 2:1 binding model (red) while for ALK a 1:1 model was used. Start and end concentrations of the 3-fold dilution series used for the WT measurements is shown as an inset while for all mutants a 3-fold dilution series from 6.4 µM-400nM was used.
   c, BLI response curves for the interaction of the site 2 ALKAL1^{F76E} mutant with LTK_{TG-EGFL}.
   d, BLI response curves for the interaction of the site 2 ALKAL2^{F97E} and ALKAL2^{H100A} with LTK_{TG-EGFL}.
   e, BLI response curves for the interaction of the site 2 ALKAL2^{F97E} with ALK_{TG-EGFL}.
   f, SDS-PAGE analysis of purified ALKAL1 and ALKAL2 mutants used in Ba/F3 and SEC-MALLS assays.
   g, Western blot analysis of phosphorylated ALK (Y1278 and Y1604) after stimulation with ALKAL2^{WT}, ALKAL2^{R123E/R136E}, ALKAL2^{F97E} and ALKAL2^{H100A}.
   h, Capacity of ALKAL1 and ALKAL2 mutants to form complexes with LTK_{TG-EGFL} and ALK_{TG-EGFL} respectively as characterized by SEC-MALLS. Differential refractive index (left axis) is plotted against the determined molecular weight (right axis). LTK_{TG-EGFL} (orange trace), LTK_{TG-EGFL}-ALKAL1^{R102E/R115E} (green trace) and LTK_{TG-EGFL}-ALKAL1^{F76E} (blue trace).
   ALK_{TG-EGFL} (pink trace), ALK_{TG-EGFL}-ALKAL2^{R123E/R136E} (green trace) and ALK_{TG-EGFL}-ALKAL2^{F97E} (blue trace). The ALKAL1 site 1 mutant is unable to form a complex with ALK while the site 2 mutant is still forms a binary complex.
   i, Capacity of the LTK_{TG-EGFL}^{R241A} LTK_{TG-EGFL}^{R241A/N369G} site 3 mutants to form complexes with ALKAL1 as characterized by SEC-MALLS. LTK_{TG-EGFL}^{R241A} (red trace), LTK_{TG-EGFL}^{R241A/N369G} (cyan trace), LTK_{TG-EGFL}^{R241A}-ALKAL1 (green trace), LTK_{TG-EGFL}^{R241A/N369G}-ALKAL1 (blue trace). LTK_{TG-EGFL} (orange trace) and LTK_{TG-EGFL}-ALKAL1 (pink trace) are shown for comparison.
   j, Cell proliferation of Ba/F3 cells expressing ALK^{WT} or ALK^{M751T} upon stimulation with 50 nM ALKAL1 or 50 nM ALKAL2. Western blot analysis of ALK^{WT} or ALK^{M751T} expression is shown as an inset.

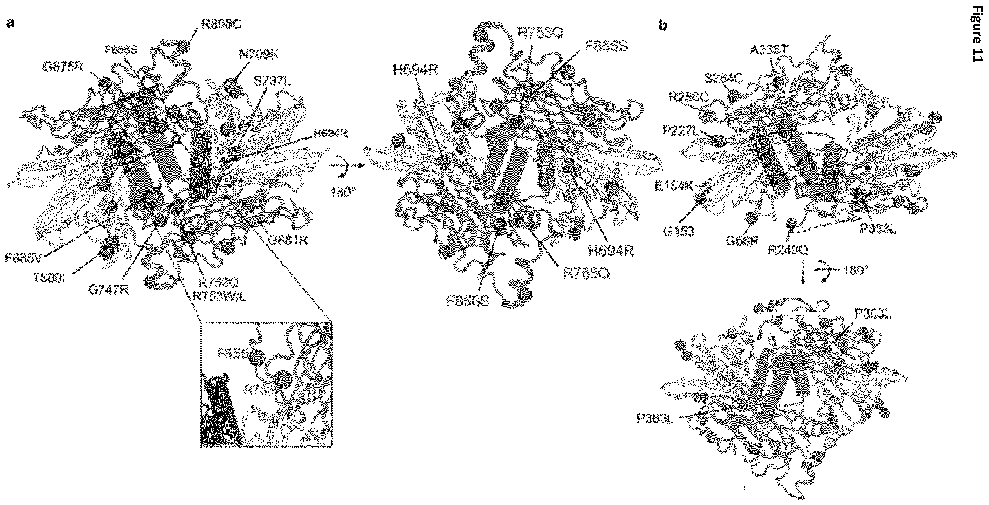

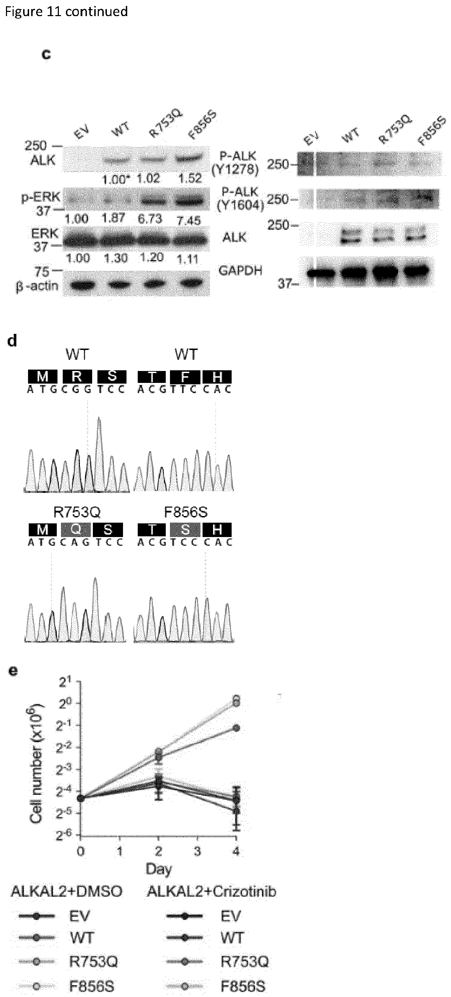

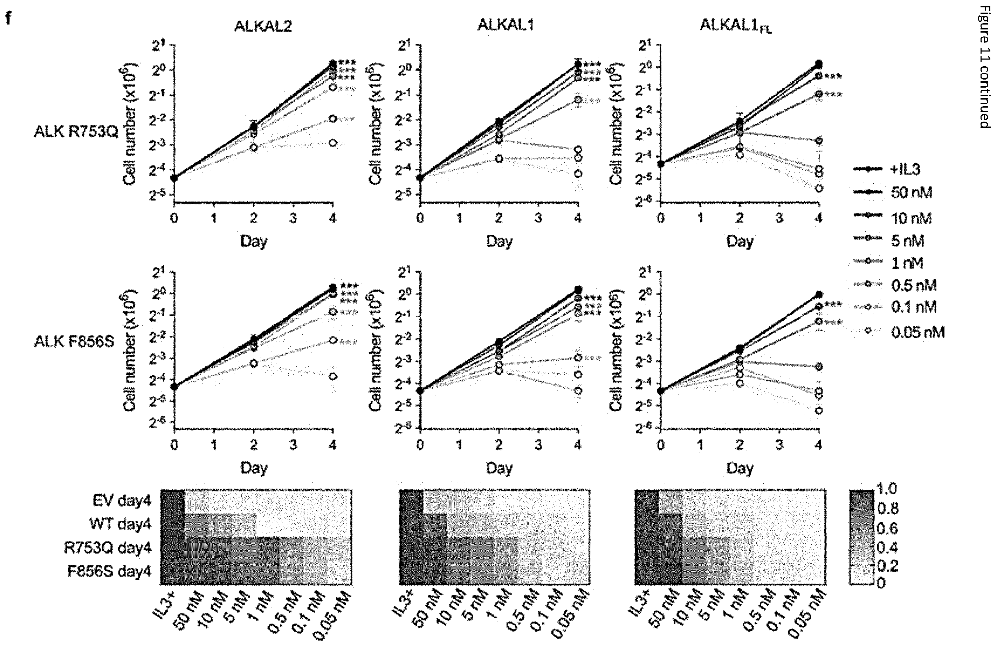

Figure 11. Mapping of missense mutations on the structures of the ALK-ALKAL2 and LTK-ALKAL1 complexes.
   a, Mapping of most frequent SNPs (GnomAD) to the ALKAL2-ALK_{TG} complex shown in top view. SNPs also found in COSMIC database (https://cancer.sanger.ac.uk/cosmic) are also indicated on the bottom view. Mutations further characterized in this study are coloured red. Inset shows the detailed position of R753 in the ALK_{TG}-ALKAL2complex.
   b, Mapping of most frequent SNPs (GnomAD) to the ALKAL1-LTK_{TG} complex shown in top view. SNPs also found in COSMIC database (https://cancer.sanger.ac.uk/cosmic) are also indicated on the bottom view.
   c, Western blot analysis of the expression levels of ALK^{WT} and the ALK^{R753Q} and ALK^{F856S} mutants and their ERK phosphorylation (left). On the right-side western blot analysis of phosphorylated ALK^{WT}, ALK^{R753Q} and ALK^{F856S} is shown. Representative results from three biologically independent experiments with similar results.
   d, Sanger sequencing of cDNA showing WT or mutant ALK expression in isogenic Ba/F3 cells.
   e, Cell proliferation of ALK expressing Ba/F3 cells treated with 10mM ALKAL2 with and without addition of crizotinib for ALK R753Q and F856S mutants. Data for EV and WT ALK are repeated from Figure 7 for direct comparison. Crizotinib is also able to inhibit ALKAL2 induced proliferation for mutant ALK, indicating ALK dependent signalling. (n = 3 biologically independent experiments; mean ± s.d.; two-way ANOVA with Tukey's multiple comparison test compared with DMSO control).
   f, Cell proliferation of Ba/F3 cells expressing ALK carrying the R753Q or F856S mutation upon stimulation by a concentration series of ALKAL2, ALKAL1 or ALKAL1_{FL} at indicated concentration. (n = 3 biologically independent experiments; mean ± s.d.; two-way ANOVA with Tukey's multiple comparison test compared with ALK^{WT}. ALKAL-induced cell growth relative to that of cells cultured with IL-3 is shown in a heatmap representation. EV and ALK^{WT} controls are included for comparison. **P* < 0.05, ***P <* 0.01 and ****P* < 0.001.

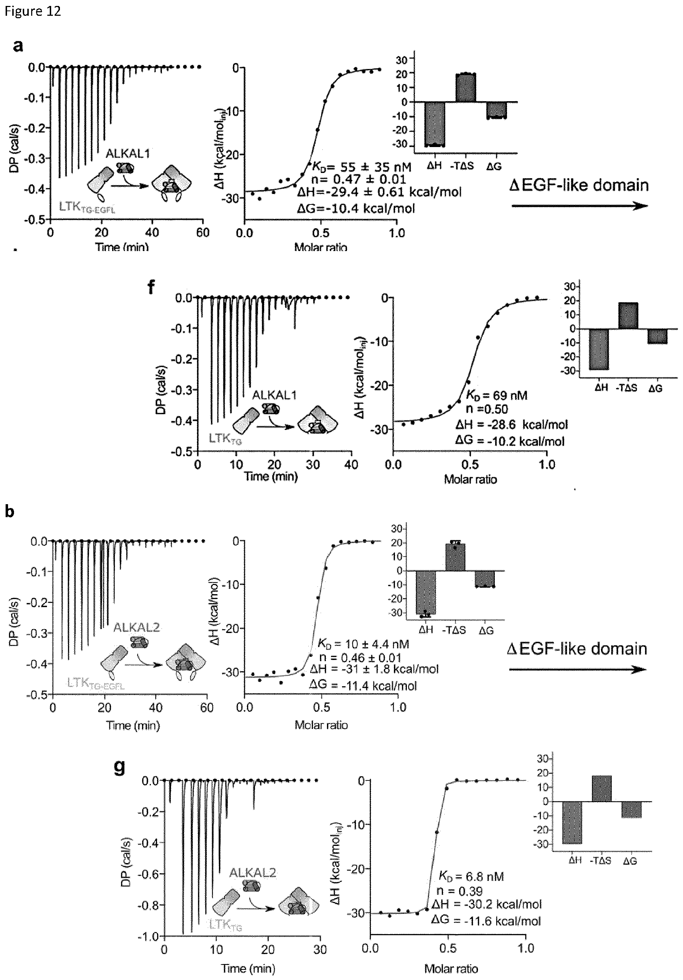

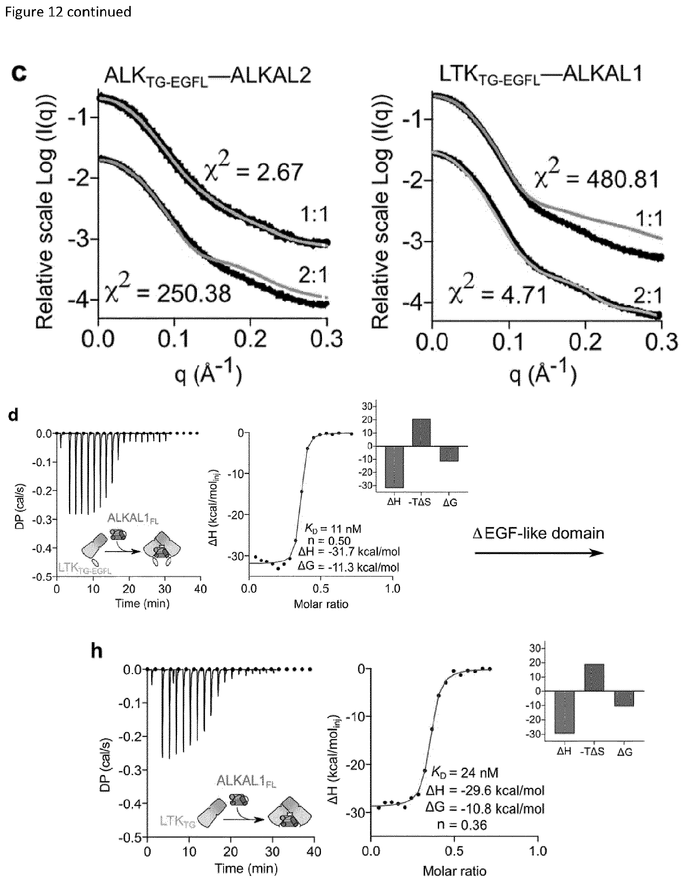

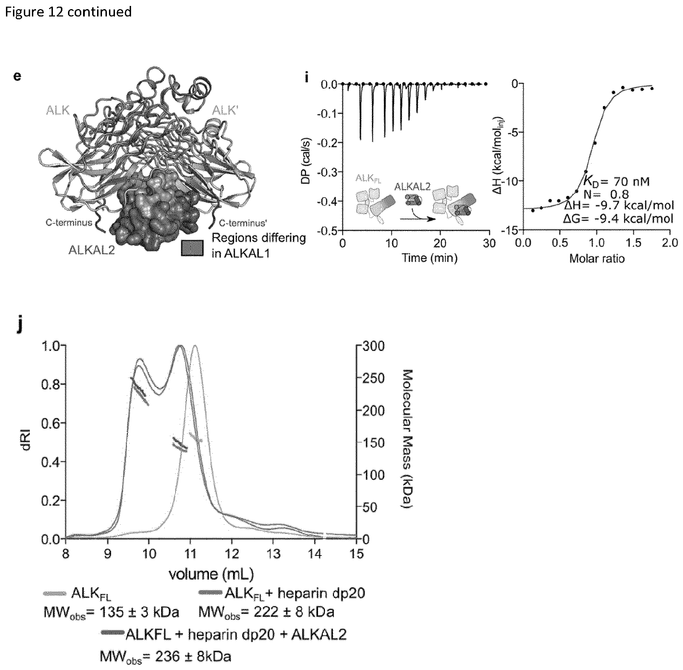

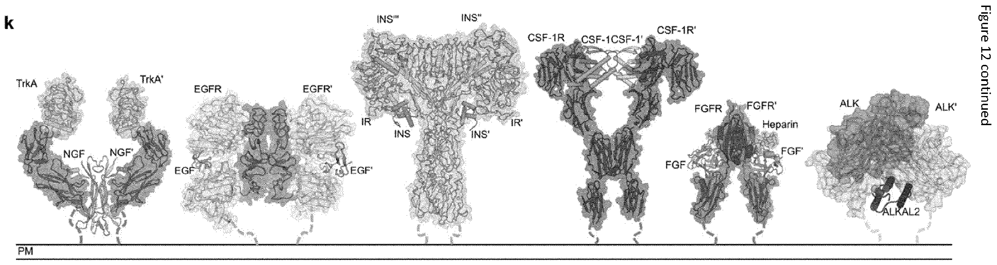

Figure 12. Mechanistic insights for the assembly of ALK/LTK-cytokine complexes derived from microcalorimetry, SAXS, and SEC-MALLS.
   a, ITC experiments for the titration of LTK_{TG-EGFL} (5 µM) with ALKAL1 (56 µM).
   b, Titration of LTK_{TG-EGFL} (12 µM) with ALKAL2 (56 µM).
   ITC titration curves, the left panel shows the raw data with the differential electrical power (DP) plotted against time. The right panel represents the binding isotherm obtained from the integration of the raw data and fitted to a one-site model. Standard deviations were calculated based on 3 measurements.
   c, Small-angle X-ray scattering analysis and calculated FoXS fits of the binary ALK_{TG-EGFL}-ALKAL2 (pink), ternary ALK_{TG-EGFL}-ALKAL2 (purple), binary LTK_{TG-EGFL}-ALKAL1 (orange) and ternary LTK_{TG-EGFL}-ALKAL1 (light orange) to experimental SAXS data (black curves).
   d, ITC experiments for the titration of LTK_{TG-EGFL} (7.2 µM) with ALKAL1_{FL} (55 µM)
   e, Ternary ALK_{TG}:ALKAL2 complex with regions differing with ALKAL1 coloured vermillion. C-termini of the TG domains leading towards the EGF-like domains are coloured red.
   f, ITC experiments for the titration of LTK_{TG} (10 µM) with ALKAL1 (70 µM) Standard deviations were calculated based on 2 measurements.
   g, ITC experiments for the titration of LTK_{TG} (10 µM) with ALKAL2 (100 µM) Standard deviations were calculated based on 2 measurements.
   h, ITC experiments for the titration of LTK_{TG} (10 µM) with ALKAL1_{FL} (40 µM) Standard deviations were calculated based on 2 measurements.
   i, ITC experiments for the titration of ALK_{FL} (8 µM) with ALKAL2 (82 µM) Standard deviations were calculated based on 2 measurements.
   j, Characterization of heparin induced ALK_{FL} dimerization by SEC-MALLS. Differential refractive index (left axis) is plotted against the determined molecular weight (right axis). ALK_{FL} (pink), ALK_{FL} complexes with heparin dp20 (green) and ALK_{FL} complexed with heparin dp20 and ALKAL2 (blue trace).
   k, Overview of the different ligand-mediated extracellular assemblies across RTKs. Trka-NGF (PDB: 2IFG), EGFR-EGF (PDB: NIVO), INSR-INS (PDB: 6PXW), CSF1R-CSF1 (PDB: 4WRM), FGFR-heparin-FGF (PDB: 1FQ9), ALK_{TG}-ALKAL2 (PDB: 7NWZ).

### Detailed description of the invention

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., current Protocols in Molecular Biology (Supplement 100), John Wiley & Sons, New York (2012), for definitions and terms of the art. Unless defined otherwise, all technical and scientific terms used herein have the same meaning as commonly understood by one of ordinary skill in the art (e.g. in molecular biology, biochemistry, structural biology, and/or computational biology).

Anaplastic lymphoma kinase (ALK) and the related leukocyte tyrosine kinase (LTK) are recently deorphanized receptor tyrosine kinases (RTK) involved in neural development, cancer, and autoimmune diseases^{1,2}. Furthermore, ALK has emerged as a surprising key regulator of energy expenditure and weight gain through signaling in the hypothalamus³. Despite such pleiotropy in physiology and disease, structural insights into ALK and LTK and their complexes with cognate cytokines had remained elusive. Here, we show that the ALKAL cytokine-binding segments of ALK and LTK comprise an unprecedented architectural chimera of a permuted TNF-like module that braces a Glycine-rich subdomain featuring a hexagonal lattice of long poly-glycine-ll helices. The cognate ALKAL cytokines are monomeric, asymmetric three-helix bundles with strikingly open structures. Yet, cytokine-mediated homodimerization of ALK and LTK leads to receptor-receptor contacts with twofold symmetry that fully tent a single cytokine molecule near the cell membrane via distinct cytokine-receptor interfaces. We show that the apparent cytokine preferences of ALK and LTK are dictated by their membrane-proximal EGF-like domains. Assisted by diverse structure-function findings, in the present invention a structural and mechanistic blueprint for the extracellular complexes of ALK/LTK family receptors is provided, thereby completing the repertoire of cytokine-driven dimerization mechanisms adopted by human RTK. Accordingly, in a first embodiment the present invention provides compositions in crystalline form selected from the complex i) ALK_{TG} depicted in SEQ ID NO:1, interacting with ALKAL2, depicted in SEQ ID NO:2, and the complex ii) LTK_{TG} depicted in SEQ ID NO:3, interacting with ALKAL1 depicted in SEQ ID NO:4, and Nb3.16, depicted in SEQ ID NO:5, characterized in that the crystals are:
i) a crystal between ALK_{TG} depicted in SEQ ID NO:1, interacting with ALKAL2, depicted in SEQ ID NO:2, in the space group P43212, with the following crystal lattice constants: a=97.57 Å ± 5%, b=97.57 Å ± 5%, c=355.35 Å ± 5%, α=β=γ=90°, and
ii) a crystal between LTK_{TG} depicted in SEQ ID NO:3, interacting with ALKAL1 depicted in SEQ ID NO:4, and Nb3.16, depicted in SEQ ID NO:5, in the space group P61, with the following crystal lattice constants: a=129.11 Å ± 5%, b=129.11 Å ± 5%, c=109.75 Å ± 5%, α=90°, β=90°, γ=120°. The wording "interacting with" is equivalent to "bound with, or bound to, or bounding with, or bounding to" or "interacted to". Thus, ALK_{TG} (SEQ ID NO: 1) interacting with ALKAL2 (SEQ ID NO: 2) can also be written as ALK_{TG} (SEQ ID NO: 1) - ALKAL2 (SEQ ID NO: 2) and ii) LTK_{TG} (SEQ ID NO: 3) interacting with ALKAL1 (SEQ ID NO: 4) and Nb3.16 (SEQ ID NO: 5) can be written as LTK_{TG} (SEQ ID NO: 3) - ALKAL1 (SEQ ID NO: 4) - Nb3.16 (SEQ ID NO: 5).

In a specific embodiment the compositions have a three-dimensional structure wherein the crystal i) comprises an atomic structure characterized by the coordinates depicted in 7NWZ and wherein the crystal ii) comprises an atomic structure characterized by the coordinates depicted in 7NX0. 7NWZ and 7NXO are the ID numbers of the structures present in the PDB database also known as the RSCB Protein databank and available on internet on https://www.rcsb.org). These ID numbers are also publicly available in De Munck S et al (2021) Nature, Vol 600, starting on page 143 - see Extended Data Table 1 in this article reference.

In yet another embodiment the invention provides a computer-assisted method of identifying, designing or screening for a compound that can potentially interact with a crystal selected from a crystal i) or ii) as defined herein before, comprising performing structure-based identification, design or screening of a compound based on the compound's interactions with a structure defined by the atomic coordinates as defined herein before.

In yet another embodiment the invention provides a method for identifying a compound that can bind to the complex i) ALK_{TG} - ALKAL2 or to the complex ii) LTK_{TG} - ALKAL1 - Nb3.16, comprising dipping candidate small molecule compounds with the complex ALK_{TG} - ALKAL2 or the complex LTK_{TG} - ALKAL1-Nb3.16L, and allowing co-crystallization, and screening candidate agonists or antagonists by using a method for measuring intermolecular interaction and comparing, designing and docking the 3D structures i) or ii) as defined herein and a candidate ligand by computer modeling.

Disclosed herein is a method of identifying, designing or screening for a compound that can interact with the complex i) ALK_{TG} - ALKAL2 or to the complex ii) LTK_{TG} - ALKAL1 - Nb3.16, including performing structure-based identification, design, or screening of a compound based on the compound's interactions with the complex i) ALK_{TG} - ALKAL2 or to the complex ii) LTK_{TG} - ALKAL1 - Nb3.16.

In yet another embodiment the invention provides a method for identifying an agonist or antagonist compound interacting with the complex i) ALK_{TG} - ALKAL2 or with the complex ii) LTK_{TG} - ALKAL1- Nb3.16 comprising an entity selected from the group consisting of an antibody, a peptide, a non-peptide molecule and a chemical compound, wherein said compound is capable of enhancing or disrupting the interaction of the bound entities (or molecules) of the complex i) ALK_{TG} - ALKAL2 or the interaction of the bound entities (or molecules) complex ii) LTK_{TG} - ALKAL1 - Nb3.16 wherein said process includes:
i) introducing into suitable computer program parameters defining an interacting surface based on the conformation of the complex i) ALK_{TG} - ALKAL2 or the complex ii) LTK_{TG} - ALKAL1 - Nb3.16 corresponding to the atomic coordinates of 7NWZ and 7NX0, wherein said program displays a three-dimensional model of the interacting surface,
ii) creating a three-dimensional structure of a test compound in said computer program;
iii) displaying a superimposing model of said test compound on the three-dimensional model of the interacting surface;
iv) and assessing whether said test compound model fits spatially into an interaction site.

As used herein the term "homologue" means a protein having at least 80% amino acid sequence identity with human ALK, LTK, ALKAL1 or ALKAL2. Preferably, the percentage identity is 85, 90%, 95% or higher. As used herein, the term "crystal" means a structure (such as a three-dimensional (3D) solid aggregate) in which the plane faces intersect at definite angles and in which there is a regular structure (such as an internal structure) of the constituent chemical species. The term "crystal" refers in particular to a solid physical crystal form such as an experimentally prepared crystal.

Details about the crystal structures (the complexes i) and ii) as described herein) are depicted in Table 1.

Crystals may be constructed with the wild-type ALK_{TG}, ALKAL2 or wild type LTK_{TG}, ALKAL1 polypeptides or variants thereof, including allelic variants and naturally occurring mutations as well as genetically engineered variants. Typically, variants have at least 90%, at least 95% sequence identity with a corresponding wild-type polypeptide. In the crystals of the invention, the polypeptides are human. Alternatively, the polypeptides are from dog, cat, swine, horse, chicken.

SEQ ID NO: 6 depicts the amino acid sequence of the human ALK tyrosine kinase receptor (herein abbreviated as ALK).

SEQ ID NO: 1 (herein abbreviated as ALK_{TG}) is the sequence of amino acids 648 to 985 of the sequence depicted in SEQ ID NO: 6.

SEQ ID NO: 7 depicts the amino acid sequence of the human leukocyte tyrosine kinase receptor (herein abbreviated as LTK).

SEQ ID NO: 3 (LTK_{TG}) is the sequence of amino acids 63 to 379 of the sequence depicted in SEQ ID NO: 7. SEQ ID NO: 2 depicts the amino acid sequence of human ALKAL1.

SEQ ID NO: 4 depicts the amino acid sequence of human ALKAL2.

SEQ ID NO: 5 depicts the amino acid sequence of Nb3.16.

As used herein, the term "atomic coordinates" or "set of coordinates" refers to a set of values which define the position of one or more atoms with reference to a system of axes. It will be understood by those skilled in the art that the atomic coordinates may be varied, without affecting significantly the accuracy of models derived therefrom.

It will be understood that any reference herein to the atomic coordinates or subset of the atomic coordinates shown in 7NWZ and 7NX0 present in the on line RCSB protein database shall include, unless specified otherwise, atomic coordinates having a root mean square deviation of backbone atoms of not more than 1.5 Å, preferably not more than 1 Å, when superimposed on the corresponding backbone atoms described by the atomic coordinates shown in 7NWZ or 7NX0 present in the on line RCSB protein database.

The following defines what is intended by the term "root mean square deviation ('RMSD')" between two data sets. For each element in the first data set, its deviation from the corresponding item in the second data set is computed. The squared deviation is the square of that deviation, and the mean squared deviation is the mean of all these squared deviations. The root mean square deviation is the square root of the mean squared deviation.

In a preferred embodiment, the crystals have the atomic coordinates as shown in 7NWZ and 7NX0 present in the on line RCSB protein database.

Further, it will be appreciated that a set of atomic coordinates for one or more polypeptides is a relative set of points that define a shape in three dimensions. Thus, it is possible that an entirely different set of coordinates could define a similar or identical shape. Moreover, slight variations in the individual coordinates will have little effect on overall shape. The variations in coordinates may be generated due to mathematical manipulations of the atomic coordinates. For example, the atomic coordinates set forth in 7NWZ and 7NX0 present in the on line RCSB protein database could be manipulated by crystallographic permutations of the atomic coordinates, fractionalization of the atomic coordinates, integer additions or subtractions to sets of the structure coordinates, inversion of the atomic coordinates, special labeling of amino acids, polypeptide chains, heteroatoms, ligands, solvent molecules, or combinations thereof.

Alternatively, modification in the crystal structure due to mutations, additions, substitutions, and/or deletions of amino acids, or other changes in any of the components that make up the crystal could also account for variations in atomic coordinates.

Various computational analyses are used to determine whether a molecular complex or a portion thereof is sufficiently similar to all or parts of the structure of the complex i) ALK_{TG} - ALKAL2 or with the complex ii) LTK_{TG} - ALKAL1 - Nb3.16. Such analyses may be carried out in available software applications which are known to the skilled person. For example, a molecular similarity program permits comparisons between different structures, different conformations of the same structure, and different parts of the same structure. Comparisons typically involve calculation of the optimum translations and rotations required such that the root mean square deviation of the fit over the specified pairs of equivalent atoms is an absolute minimum. This number is given in Angstroms (Å). Accordingly, atomic coordinates of the complex i) ALK_{TG} - ALKAL2 or the complex ii) LTK_{TG} - ALKAL1 - Nb3.16 of the present invention include atomic coordinates related to the atomic coordinates listed in 7NWZ and 7NX0 present in the on line RCSB protein database by whole body translations and/or rotations. Accordingly, RMSD values listed above assume that at least the backbone atoms of the structures are optimally superimposed which may require translation and/or rotation to achieve the required optimal fit from which to calculate the RMSD value. A three- dimensional structure of the complex i) ALK_{TG} - ALKAL2 or the complex ii) LTK_{TG} - ALKAL1 - Nb3.16 or a region thereof which substantially conforms to a specified set of atomic coordinates can be modelled by a suitable modelling computer program, using information, for example, derived from the following data: (1) the amino acid sequence of the polypeptides of the complex i) ALK_{TG} - ALKAL2 or the complex ii) LTK_{TG} - ALKAL1 - Nb3.16; (2) the amino acid sequence of the related portion(s) of the protein represented by the specified set of atomic coordinates having a three-dimensional configuration; and (3) the atomic coordinates of the specified three-dimensional configuration. A three-dimensional structure of the polypeptides of the complex which substantially conforms to a specified set of atomic coordinates can also be calculated by a method such as molecular replacement, which is described in detail below.

Atomic coordinates are typically loaded onto a machine-readable medium for subsequent computational manipulation. Thus, models and/or atomic coordinates are advantageously stored on machine-readable media, such as magnetic or optical media and random-access or read-only memory, including tapes, diskettes, hard disks, CD-ROMs and DVDs, flash memory cards or chips and servers. Typically, the machine is a computer. The atomic coordinates may be used in a computer to generate a representation, e.g. an image of the three-dimensional structure of polypeptides of the complex which can be displayed by the computer and/or represented in an electronic file. The atomic coordinates and models derived therefrom may be used for a variety of purposes such as drug discovery, biological reagent (binding protein) selection and X-ray crystallographic analysis of other protein crystals.

### Molecular Replacement

The structure coordinates of the polypeptide such as those set forth in 7NWZ and 7NX0 present in the on line RCSB protein database or a subset thereof, can also be used for determining the three-dimensional structure of a distant crystallized polypeptide of the complex ALK_{TG} - ALKAL2 or the complex ii) LTK_{TG} - ALKAL1 (e.g. derived from another species such as a relevant veterinary species including cat, dog, swine, horse and chicken). This may be achieved by any of a number of well-known techniques, including molecular replacement. Methods of molecular replacement are generally known by those skilled in the art. Generally, molecular replacement involves the following steps: i) X-ray diffraction data are collected from the crystal of a crystallized target structure, then ii) the X-ray diffraction data are transformed to calculate a Patterson function, then iii) the Patterson function of the crystallized target structure is compared with a Patterson function calculated from a known structure (referred to herein as a search structure or search model) , iv) the Patterson function of the search structure is rotated on the target structure Patterson function to determine the correct orientation of the search structure in the crystal to obtain a rotation function, v) a translation function is then calculated to determine the location of the search structure with respect to the crystal axes. Alternatively, likelihood-based molecular replacement methods can be used to determine the location of the search structure. Once the search structure has been correctly positioned in the unit cell, initial phases for the experimental data can be calculated. These phases are necessary for calculation of an electron density map from which structural features and differences can be observed to allow construction of a molecular model and refinement of the structure. Preferably, the structural features (e.g., amino acid sequence, conserved disulphide bonds, and beta-strands or beta-sheets) of the search molecule are related to the crystallized target structure. The electron density map can, in turn, be subjected to any well-known model building and structure refinement techniques to provide a final, accurate structure of the unknown (i.e. target) crystallized molecular structure. Obtaining accurate values for the phases, by methods other than molecular replacement, is often a time-consuming process that involves iterative cycles of approximations and refinements and greatly hinders the solution of crystal structures. However, when the crystal structure of a protein containing at least a homologous portion has been solved, the phases from the known structure provide a satisfactory starting estimate of the phases for the unknown structure. By using molecular replacement, all or part of the structure coordinates of the polypeptides of the complexes as described herein (and set forth in 7NWZ or 7NX0 present in the on line RCSB protein database) can be used to determine the structure of another crystallized complex (ALK_{TG} - ALKAL2 or the complex ii) LTK_{TG} - ALKAL1) whose structure is unknown, more rapidly and more efficiently than attempting to determine such information *ab initio.*

The structure of any portion of any crystallized molecular ALK_{TG} - ALKAL2 complex or the complex ii) LTK_{TG} - ALKAL1 that is sufficiently homologous to any portion of the human ALK_{TG} - ALKAL2 complex or the complex ii) LTK_{TG} - ALKAL1 can be solved by this method.

Such structure coordinates are also particularly useful to solve the structure of crystals of ALK_{TG} - ALKAL2 complex or the complex ii) LTK_{TG} - ALKAL1 co-complexed with a variety of molecules, such as chemical entities. For example, this approach enables the determination of the optimal sites for the interaction between chemical entities, and the interaction of candidate disrupting entities of the complexes or agonists of the complexes.

### Design, Selection, Fitting and Assessment of Chemical Entities that bind the ALK_{TG} - ALKAL2 complex or the complex ii) LTK_{TG} - ALKAL1

Using a variety of known modelling techniques, the crystal structures of the present invention can be used to produce models for evaluating the interaction of compounds with the complexes described herein. As used herein, the term "modelling" includes the quantitative and qualitative analysis of molecular structure and/or function based on atomic structural information and interaction models. The term "modelling" includes conventional numeric-based molecular dynamic and energy minimization models, interactive computer graphic models, modified molecular mechanics models, distance geometry and other structure-based constraint models. Molecular modelling techniques can be applied to the atomic coordinates of the complexes or parts thereof to derive a range of 3D models and to investigate the structure of binding sites, such as the binding sites with compounds. These techniques may also be used to screen for or design small and large chemical entities which are capable of binding the complexes and modulate the interaction of the elements of the complexes, in particular the disruption of the complexes. Such a screen may employ a solid 3D screening system or a computational screening system. Such modelling methods are to design or select chemical entities that possess stereochemical complementary to identified binding sites between the individual elements in the complexes. By "stereochemical complementarity" it is meant that the compound makes a sufficient number of energetically favourable contacts with individual elements in the complexes as to have a net reduction of free energy on binding to these individual elements in the complexes. By "stereochemical similarity" we mean that the compound makes about the same number of energetically favourable contacts with the complexes set out by the coordinates shown in 7NWZ and 7NX0 present in the on line RCSB protein database. In addition, modelling methods may also be used to design or select chemical entities that possess stereochemical complementarity to the complexes of the invention. By stereochemical complementarity it is meant that the compound makes energetically favourable contacts with the complexes as defined by coordinates shown in 7NWZ and 7NX0 present in the on line RCSB protein database. By "match" we mean that the identified portions interact with the surface residues, for example, via hydrogen bonding or by non-covalent van der Waals and Coulomb interactions (with surface or residue) which promote dissolvation of the molecule within the site, in such a way that retention of the molecule at the binding site is favoured energetically. It is preferred that the stereochemical complementarity is such that the compound has a K_{d} for the binding site of less than 10⁻⁴M, more preferably less than 10⁻⁵M and more preferably 10⁻⁶M. In a most preferred embodiment, the K_{d} value is less than 10⁻⁸M and more preferably less than 10⁻⁹M.

Chemical entities which are complementary to the shape and electrostatics or chemistry of the complexes characterized by amino acids positioned at atomic coordinates set out in 7NWZ and 7NX0 present in the on line RCSB protein database will be able to bind to the complexes, and when the binding is sufficiently strong, substantially inhibit the interaction of the individual elements in the complexes.

A number of methods may be used to identify chemical entities possessing stereochemical and structural complementarity to the structure or substructures of the complexes. For instance, the process may begin by visual inspection of a selected binding site in the complexes on the computer screen based on the coordinates in 7NWZ and 7NX0 present in the on line RCSB protein database generated from the machine-readable storage medium. Alternatively, selected fragments or chemical entities may then be positioned in a variety of orientations, or docked, within the selected binding site. Modelling software is well known and available in the art. This modelling step may be followed by energy minimization with standard available molecular mechanics force fields. Once suitable chemical entities or fragments have been selected, they can be assembled into a single compound. In one embodiment, assembly may proceed by visual inspection of the relationship of the fragments to each other on the three-dimensional image displayed on a computer screen in relation to the structure coordinates of selected binding sites in the complexes. This is followed by manual model building, typically using available software. Alternatively, fragments may be joined to additional atoms using standard chemical geometry. The above-described evaluation process for chemical entities may be performed in a similar fashion for chemical compounds.

Databases of chemical structures are available from a number of sources including Cambridge Crystallographic Data Centre (Cambridge, U.K.), Molecular Design, Ltd., (San Leandro, Calif.), Tripos Associates, Inc. (St. Louis, Mo.), Chemical Abstracts Service (Columbus, Ohio), the Available Chemical Directory (Symyx Technologies, Inc.), the Derwent World Drug Index (WDI), BioByteMasterFile, the National Cancer Institute database (NCI), Medchem Database (BioByte Corp.), and the Maybridge catalogue. Once an entity or compound has been designed or selected by the above methods, the efficiency with which that entity or compound may bind to the complexes can be tested and optimised by computational evaluation. For example, a compound that has been designed or selected to function as binding compound to the complexes must also preferably traverse a volume not overlapping that occupied by the binding site when it is bound to the native complexes. An effective complex binding compound must preferably demonstrate a relatively small difference in energy between its bound and free states (i.e. a small deformation energy of binding). Thus, the most efficient complex binding compound should preferably be designed with a deformation energy of binding of not greater than about 10 kcal/mole, preferably, not greater than 7 kcal/mole. Complex binding compounds may interact with the complexes in more than one conformation that are similar in overall binding energy. In those cases, the deformation energy of binding is taken to be the difference between the energy of the free compound and the average energy of the conformations observed when the compound binds to the protein. Further, a compound designed or selected as binding to a complex may be further computationally optimised so that in its bound state it would preferably lack repulsive electrostatic interaction with the target protein.

Once a binding compound to the complexes has been optimally selected or designed, as described above, substitutions may then be made in some of its atoms or side groups to improve or modify its binding properties. Generally, initial substitutions are conservative, i.e. the replacement group will have approximately the same size, shape, hydrophobicity and charge as the original group. It should, of course, be understood that components known in the art to alter conformation should be avoided. Such substituted chemical compounds may then be analysed for efficiency of fit to the complexes by the same computer methods described in detail above.

Naturally, specific computer software is available in the art to evaluate compound deformation energy and electrostatic interaction. The screening/design methods may be implemented in hardware or software, or a combination of both. However, preferably, the methods are implemented in computer programs executing on programmable computers each comprising a processor, a data storage system (including volatile and non-volatile memory and/or storage elements), at least one input device, and at least one output device. Program code is applied to input data to perform the functions described above and generate output information. The output information is applied to one or more output devices, in known fashion. The computer may be, for example, a personal computer, microcomputer, or workstation of conventional design. Each program is preferably implemented in a high-level procedural or object-oriented programming language to communicate with a computer system. However, the programs can be implemented in assembly or machine language, if desired. In any case, the language may be compiled or interpreted language. Each such computer program is preferably stored on a storage medium or device (e.g., ROM or magnetic diskette) readable by a general or special purpose programmable computer, for configuring and operating the computer when the storage media or device is read by the computer to perform the procedures described herein. The system may also be considered to be implemented as a computer-readable storage medium, configured with a computer program, where the storage medium so configured causes a computer to operate in a specific and predefined manner to perform the functions described herein.

### Compounds

Compounds include both those designed or identified using a screening method of the invention and those which are capable of recognising and binding to the complexes as defined above. Compounds capable of recognising and binding to the complexes may be produced using a screening method based on use of the atomic coordinates corresponding to the 3D structure of the complexes. Compounds capable of recognising and binding to the complexes may be produced using a screening method based on the use of the atomic coordinates corresponding to the 3D structure of the complexes. The candidate compounds and/or compounds identified or designed using a method of the present invention may be any suitable compound, synthetic or naturally occurring, preferably synthetic. In one embodiment, a synthetic compound selected or designed by the methods of the invention preferably has a molecular weight equal to or less than about 5000, 4000, 3000, 2000, 1000 or 500 daltons. A compound is preferably soluble under physiological conditions. The compounds may encompass numerous chemical classes, though typically they are organic molecules, preferably small organic compounds having a molecular weight of more than 50 and less than about 2,500 daltons, preferably less than 1,500, more preferably less than 1,000 and yet more preferably less than 500. Such compounds can comprise functional groups necessary for structural interaction with proteins, particularly hydrogen bonding, and typically include at least an amine, carbonyl, hydroxyl or carboxyl group, preferably at least two of the functional chemical groups. The compound may comprise cyclical carbon or heterocyclic structures and/or aromatic or polyaromatic structures substituted with one or more of the above functional groups. Compounds can also comprise biomolecules including peptides, saccharides, fatty acids, steroids, purines, pyrimidines, derivatives, structural analogues, or combinations thereof. Compounds may include, for example: (1) peptides such as soluble peptides, including Ig-tailed fusion peptides and members of random peptide libraries and combinatorial chemistry-derived molecular libraries made of D- and/or L-configuration amino acids; (2) phosphopeptides (e.g. members of random and partially degenerate, directed phosphopeptide libraries, (3) antibodies (e.g., polyclonal, monoclonal, humanized, anti-idiotypic, chimeric, and single chain antibodies, nanobodies as well as Fab, (Fab)₂, Fab expression library and epitope-binding fragments of antibodies); (4) non-immunoglobulin binding proteins such as but not restricted to avimers, DARPins and lipocalins; (5) nucleic acid-based aptamers; and (6) small organic and inorganic molecules.

Synthetic compound libraries are commercially available from, for example, Maybridge Chemical Co. (Tintagel, Cornwall, UK), AMRI (Budapest, Hungary) and ChemDiv (San Diego, Calif.), Specs (Delft, The Netherlands). In addition, numerous means are available for random and directed synthesis of a wide variety of organic compounds and biomolecules, including expression of randomized oligonucleotides. Alternatively, libraries of natural compounds in the form of bacterial, fungal, plant and animal extracts can be readily produced. In addition, natural or synthetic compound libraries and compounds can be readily modified through conventional chemical, physical and biochemical means and may be used to produce combinatorial libraries. In another approach, previously identified pharmacological agents can be subjected to directed or random chemical modifications, such as acylation, alkylation, esterification, amidification, and the analogues can be screened for disrupting the complexes. In addition, numerous methods of producing combinatorial libraries are known in the art, including those involving biological libraries; spatially addressable parallel solid phase or solution phase libraries; synthetic library methods requiring deconvolution; the "one-bead one-compound" library method; and synthetic library methods using affinity chromatography selection. The biological library approach is limited to polypeptide or peptide libraries, while the other four approaches are applicable to polypeptide, peptide, nonpeptide oligomer, or small molecule libraries of compounds. Compounds also include those that may be synthesized from leads generated by fragment-based drug design, wherein the binding of such chemical fragments is assessed by soaking or co-crystallizing such screen fragments into crystals provided by the invention and then subjecting these to an X-ray beam and obtaining diffraction data. Difference Fourier techniques are readily applied by those skilled in the art to determine the location within the complex structure at which these fragments bind, and such fragments can then be assembled by synthetic chemistry into larger compounds with increased affinity for a particular position in the complexes. Further, compounds identified or designed using the methods of the invention can be a peptide or a mimetic thereof. The isolated peptides or mimetics may be conformationally constrained molecules or alternatively molecules which are not conformationally constrained such as, for example, non-constrained peptide sequences. The term "conformationally constrained molecules" means conformationally constrained peptides and conformationally constrained peptide analogues and derivatives. In addition, the amino acids may be replaced with a variety of uncoded or modified amino acids such as the corresponding D-amino acid or N-methyl amino acid. Other modifications include substitution of hydroxyl, thiol, amino and carboxyl functional groups with chemically similar groups. With regard to peptides and mimetics thereof, still other examples of other unnatural amino acids or chemical amino acid analogues/derivatives can be introduced as a substitution or addition. Also, a peptidomimetic may be used. A peptidomimetic is a molecule that mimics the biological activity of a peptide but is no longer peptidic in chemical nature. By strict definition, a peptidomimetic is a molecule that no longer contains any peptide bonds (that is, amide bonds between amino acids). However, the term peptide mimetic is sometimes used to describe molecules that are no longer completely peptidic in nature, such as pseudo-peptides, semi-peptides and peptoids. Whether completely or partially non-peptide, peptidomimetics for use in the methods of the invention, provide a spatial arrangement of reactive chemical moieties that closely resembles the three-dimensional arrangement of active groups in the peptide on which the peptidomimetic is based. As a result of this similar active-site geometry, the peptidomimetic has effects on biological systems which are similar to the biological activity of the peptide. There are sometimes advantages for using a mimetic of a given peptide rather than the peptide itself, because peptides commonly exhibit two undesirable properties: (1) poor bioavailability; and (2) short duration of action. Peptide mimetics offer an obvious route around these two major obstacles, since the molecules concerned are small enough to be both orally active and have a long duration of action. There are also considerable cost savings and improved patient compliance associated with peptide mimetics, since they can be administered orally compared with parenteral administration for peptides. Furthermore, peptide mimetics are generally cheaper to produce than peptides. Naturally, those skilled in the art will recognize that the design of a peptidomimetic may require slight structural alteration or adjustment of a chemical structure designed or identified using the methods of the invention. In general, chemical compounds identified or designed using the methods of the invention can be synthesized chemically and then tested for ability to disrupt the complexes, using any of the methods described herein. The peptides or peptidomimetics of the present invention can be used in assays for screening for candidate compounds which bind to selected regions or selected conformations of the complexes. Binding can be either by covalent or non-covalent interactions, or both. Examples of non-covalent interactions include electrostatic interactions, van der Waals interactions, hydrophobic interactions and hydrophilic interactions.

Compounds preferably have an affinity for the complexes, sufficient to provide adequate binding for the intended purpose. Suitably, such compounds have an affinity (K_{d}) of from 10⁻⁵ to 10⁻¹⁵ M. For use as a therapeutic, the compound suitably has an affinity (K_{d}) of from 10⁻⁷ to 10⁻¹⁵ M, preferably from 10⁻⁸ to 10⁻¹² M and more preferably from 10⁻¹⁰ to 10⁻¹² M.

### Screening Assays and Confirmation of Binding

Compounds may be subjected to further confirmation of binding to the complexes and structural determination, as described herein. Compounds designed or selected according to the methods of the present invention are preferably assessed by a number of *in vitro* and *in vivo* assays of ALK and LTK function to confirm their ability to interact with and modulate the complexes, in particular the disruption of the complexes. Libraries may be screened in solution by methods generally known in the art for determining whether ligands competitively bind at a common binding site. Such methods may include screening libraries in solution, or on beads or chips. Where the screening assay is a binding assay, polypeptides of the complexes, may be joined to a label, where the label can directly or indirectly provide a detectable signal. Various labels include radioisotopes, fluorescent molecules, chemiluminescent molecules, enzymes, specific binding molecules, particles, e.g., magnetic particles, and the like. Specific binding molecules include pairs, such as biotin and streptavidin, digoxin and antidigoxin, etc. For the specific binding members, the complementary member would normally be labelled with a molecule that provides for detection, in accordance with known procedures. A variety of other reagents may be included in the screening assay. These include reagents like salts, neutral proteins, e.g., albumin, detergents, etc., which are used to facilitate optimal protein-protein binding and/or reduce non-specific or background interactions. Reagents that improve the efficiency of the assay, such as protease inhibitors, nuclease inhibitors, antimicrobial agents, etc., may be used. The components are added in any order that produces the requisite binding. Incubations are performed at any temperature that facilitates optimal activity, typically between 4°C and 40°C. Direct binding of compounds to the complexes can also be done for example by Surface Plasmon Resonance (BIAcore).

**Table 1: Crystallographic data and refinement statistics**

| | ALK_{TG-EGFL} | ALK_{TG-EGFL}-Fab324 | LTK_{TG} | ALK_{TG}-ALKAL2 | LTK_{TG}-ALKAL1-Nb3.16 | Fab324 |
|---|---|---|---|---|---|---|
| Crystallization conditions | 0.1M Gly-Gly/AMPD pH 8.5 | 40mMPolyamines, 0.1M Gly-Gly/AMPD pH8.5 | MOPSO/Bis-Tris pH 6.3 | 0.1M MES pH 6.5 | MOPSO/BIS-TRIS pH 6.5 | 1.5M (NH₄)₂SO₄ 40 mM Glycine pH 9.5 |
| | 15% w/v PEG 3000 | | 12% PEG 20000 | | 13% PEG 8000 | |
| | 20% v/v 1,2,4-Butanetriol | 12.5% w/v PEG4000 | 25% Trimethyl propane | | 22% w/v 1,5-pentanediol | |
| | | 20% w/v 1,2,6-Hexanetriol | | 15% w/v PEG 6000 | | |
| | 1% w/v NDSB 256 | | 2% w/v NDSB 195 | 5% w/v MPD | 5mM Na₂CrO₄.4H₂O | |
| | 5mM NiCl₂.6H₂O | | 5mM YCl₃.6H₂O | | 5mM Na₂MoO₄.4H₂O | |
| | 5mM CoCl₂.6H₂O | | 5mM ErCl₃.6H₂O | | 5mM Na₂WO₄.4H₂O | |
| | 5mM ZnCl₂.6H₂O | | 5mM TbCl₃.6H₂O | | 5mM Na₂VO₄.4H₂O | |
| | 5mM MnCl₂.6H₂O | | 5mM YbCl₃.6H₂O | | | |
| Cryoprotectant | 25% v/v 1,2,4-Butanetriol | 25% w/v 1,2,6-Hexanetriol | 25% Trimethyl propane | 22% ethylene glycol | 25% 1,5-pentanediol | Saturated (NH₄)₂SO₄ |

| **Data collection** | | | | | | |
|---|---|---|---|---|---|---|
| X-ray source (beamline) | PETRAIII (P14) | ESRF (ID-23-2) | PETRAIII (P13) | SOLEIL (Proxima 2A) | SOLEIL (Proxima 2A) | PETRAIII (P13) |
| Wavelength (Å) | 1.033 | 0.8731 | 0.9763 | 0.9800 | 0.9800 | 0.9762 |
| Space group | *141* | *P 21 21 21* | *P21* | *P 43 21 2* | *P 61* | *P 21 21 21* |
| Cell dimensions | | | | | | |
| *a, b, c* (Å) | 84.93 84.93 148.67 | 109.7 137.47 144.26 | 55.04 54.46 55.01 | 97.57 97.57 355.35 | 129.11 129.11 109.75 | 81.24 87.09 126.6 |
| α,β, γ (°) | 90.00, 90.00, 90.00 | 90.00. 90.00, 90.00 | 90 111.284 90 | 90.00, 90.00, 90.00 | 90.00 90.00 120.00 | 90.00, 90.00,, 90.00 |
| Resolution (Å) | 60.05 - 3.0 (3.11 - 3.0) | 48.04 - 2.81 (2.91 - 2.81) | 51.26 - 1.3 (1.38 - 1.3) | 48.96 - 4.18 (4.32 - 4.18) | 42.3 - 1.95 (2.02 - 1.95) | 71.76 - 1.47 (1.52 - 1.47) |
| *R*ₘₑₐₛ (%) | 18.9 (114.9) | 25.4 (120.1) | 6.9 (125.5) | 20.6 (168.1) | 7.6 (147.1) | 4.2 (35.8) |
| < *I*/*σ* > | 10.15 (1.26) | 11.11 (1.91) | 11.5 (1.0) | 13.9 (2.0) | 26.6 (1.9) | 17.32 (2.4) |
| CC ½ (%) | 100 (75.8) | 99.9 (34.1) | 99.9 (54.6) | 99.9 (89.2) | 99.9 (88.5) | 99.7 (42.1) |
| Completeness (%) | 99.90 (99.90) | 99.19 (93.06) | 98.90 (98.5) | 99.50 (94.5) | 99.7 (96.3) | 96.20 (73.3) |
| Redundancy | 14.3 (14.4) | 5.8 (4.5) | 3.4 (3.2) | 25.6 (24.6) | 20.8 (19.8) | 3.8 (3.2) |
| Wilson B factor (Å²) | 104.20 | 39.46 | 14.23 | 105.89 | 38.80 | 16.20 |

| **Refinement** | | | | | | |
|---|---|---|---|---|---|---|
| Resolution (Å) | 60.05 - 3.0 | 48.04 - 2.81 | 51.26 - 1.3 | 48.78 - 4.18 | 42.3 - 1.95 | 71.76 - 1.47 |
| No. reflections | 10550 | 53379 | 145183 | 13500 | 75501 | 146924 |
| *R*_{work} / *R*_{free} (%) | 23.4/26.0 | 22.6 / 26.3 | 16.2 / 18.1 | 25.3 / 26.8 | 16.9 / 19.6 | 16.9 / 20.5 |
| No. atoms | | | | | | |
| Protein | 2496 | 10653 | 2098 | 9763 | 6502 | 6486 |
| Ligand/ion | 31 | | 46 | 98 | 4 | |
| Water | | | 232 | | 691 | 917 |
| *B*-factors (Å²) | | | | | | |
| Protein | 103.45 | 52.21 | 21.08 | 162.0 | 53.25 | 19.60 |
| Ligand/ion | 116.68 | | 25.67 | 195.3 | 116.64 | 24.54 |
| Water | | | 36.65 | | 57.86 | 30.49 |
| R.m.s. deviations | | | | | | |
| Bond lengths (Å) | 0.002 | 0.005 | 0.007 | 0.002 | 0.006 | 0.008 |
| Bond angles (°) | 0.53 | 0.87 | 1.09 | 0.50 | 0.90 | 1.03 |
| **Pdb id** | 7NX4 | 7NX3 | 7NX1 | 7NWZ | 7NX0 | 7NX2 |

Each data set was collected from a single crystal. Values in parentheses correspond to the highest resolution shell.

**Table 2: Interaction interface analysis of ALK-ALKAL2 and LTK-ALKAL1 complexes**

| List of contacting amino acids at the interfaces of the ALKAL2-ALKTG and ALKAL1-LTKTG complex. Hydrogen bonding residues were determined using the PISA server at EBI (https://www.ebi.ac.uk/msd-srv/prot_int/pistart.html) and confirmed by analysis in ChimeraX. van der Waals contacts were analyzed using the 'find contacts' function in ChimeraX. The components involved in the described interface are shown above each column. | | | | | | | | |
|---|---|---|---|---|---|---|---|---|
| LTK-ALKAL1 site 1 | | | LTK-ALKAL1 site 2 | | | LTK-ALKAL1 site 3 | | |
| ALKAL1 Chain A | LTK Chain B | dist (A) | ALKAL1 Chain A | LTK Chain C | dist (A) | LTK Chain B | LTK Chain C | dist (A) |
| **Hydrogen-bonds and salt bridges** | | | **Hydrogen-bonds and salt bridges** | | | **Hydrogen-bonds and salt bridges** | | |
| GLU 103[ OE2] | TYR 124[ OH ] | 2.57 | HIS 79[ O ] | ARG 376[ NH2] | 3.43 | ARG 241[ NE ] | GLY 88[ O ] | 3.57 |
| ARG 102[ NH2] | TYR 124[ OH ] | 3.51 | HIS 79[ NE2 ] | SER 122[ OG ] | 2.8 | ARG 241[ NH2] | GLY 88[ O ] | 3.46 |
| ARG 119[ NH2] | SER 251[ O ] | 3.28 | LYS 111[ NZ ] | TYR 124[ OH ] | 3.0 | ARG 241[ NH2] | ALA 89[ O ] | 2.86 |
| ARG 119[ NH2] | GLU 253[ OE1] | 3.09 | ARG 112[ NH2] | GLU 368[ OE1] | 3.06 | ARG 241[ NH2] | ALA 91[ O ] | 2.62 |
| ARG 119[ NE ] | GLU 253[ OE2] | 2.80 | **van der Waals contacts** | | | GLN 85[ NE2] | CYS 179[ O ] | 3.37 |
| HIS 93[ ND1] | GLN 362[ OE1] | 3.46 | | | | THR 84[ OG1] | GLU 182[ OE2] | 2.77 |
| ARG 115[ NH2] | VAL 366[ O ] | 2.89 | PHE 80 | TYR 124 | | ARG 138[ NH1] | ASN 369[ OD1] | 3.51 |
| ARG 115[ NE ] | VAL 366[ O ] | 2.84 | | PHE 143 | | ASN 369[ ND2] | ASN 369[ OD1] | 3.22 |
| ARG 112[ NH11 | 372[ OE1] | 3.59 | PRO 107 | LEU 364 | | GLY 88[ O ] | ARG 241[ NH2] | 3.16 |
| ARG 102[ NH1] | GLU GLU 372[ OE2] | 3.28 | | VAL 366 | | ALA 89[ O ] | ARG 241[ NH2] | 2.94 |
| ARG 102[ NH2] | GLU 372[ OE2] | 3.20 | ALA 108 | ALA 365 | | ALA 91[ O ] | ARG 241[ NH2] | 2.89 |
| ASN 100[ O ] | ARG 376[ NH1] | 3.61 | PHE 76 | LEU 361 | | ALA 91[ O ] | ARG 241[ NH1] | 2.86 |
| | | | | PHE 143 | | CYS 179[ O ] | GLN 85[ NE2] | 3.31 |
| **van der Waals contacts** | | | | GLN 362 | | ASN 369[ OD1] | ARG 138[ NH1] | 3.14 |
| ARG 96 | PHE 360 | | | LEU 364 | | ASN 369[ OD1] | ASN 369[ ND2] | 3.17 |
| LEU 97 | LEU 361 | | TYR 110 | LEU 364 | | **van der Waals contacts** | | |
| SER 123 | GLN 362 | | HIS 79 | LEU120 | | | | |
| GLU 103 | TYR 124 | | | PHE143 | | HIS 135 | CYS 73 | |
| LEU116 | VAL 266 | | | ARG376 | | | GLY 74 | |
| | LEU 364 | | | | | | THR 84 | |
| | PHE 143 | | | | | LEU 180 | GLN 85 | |
| LEU 120 | LEU 364 | | | | | GLU 182 | GLY 92 | |
| | GLN 362 | | | | | ARG 243 | HIS 135 | |
| | | | | | | CYS 73 | | |
| | | | | | | GLY 74 | | |
| | | | | | | THR 84 | LEU 180 | |
| | | | | | | GLN 85 | GLU 182 | |
| | | | | | | GLY 92 | ARG 243 | |
| | | | | | | | | |

| ALK-ALKAL2 site 1 | | | ALK-ALKAL2 site 2 | | | ALK-ALKAL2 site 3 | | |
|---|---|---|---|---|---|---|---|---|
| ALKAL2 Chain C | ALK Chain A | dist (A) | ALKAL2 Chain C | ALK Chain B | dist (Å) | ALK Chain A | ALK Chain B | dist (Å) |
| **Hydrogen-bonds and salt bridges** | | | **Hydrogen-bonds and salt bridges** | | | **Hydrogen-bonds and salt bridges** | | |
| TYR 739[ OH ] | ASP 134[ OE2] | 2.8 | HIS 100[ O ] | LYS 982[ NZ ] | 3.53 | THR 697[ OG1] | ILE 795[ O ] | 3.54 |
| HIS 857[ O ] | ARG 140[ NH2] | 3.90 | LYS 96[ NZ ] | TYR 966[ O ] | 3.30 | GLN 700[ N ] | ILE 795[ O ] | 3.83 |
| HIS 857[ O ] | ARG 140[ NH2] | 3.77 | HIS 100[ NE2] | SER 737[ OG ] | 3.49 | ASN 749[ ND2] | GLN 706[ OE1] | 3.20 |
| GLU 859[ OE2] | ARG 140[ NE ] | 2.96 | LYS 132[ NZ ] | GLU 978[ OE1] | 2.91 | ILE 795[ O ] | THR 697[ OG1] | 3.38 |
| TYR 966[ OH ] | ASN 121[ ND1] | 3.02 | | | | CYS 794[ O ] | GLN 700[ NE2] | 3.84 |
| VAL 972[ O ] | ARG 136[ NH2] | 3.23 | | | | ASN 749[ OD1] | GLN 706[ NE2] | 3.31 |
| GLU 974[ OE2] | ARG 136[ NE ] | 3.08 | | | | | | |
| GLU 978[ OE1] | ARG 133[ NH1] | 2.8 | | | | | | |

| **van der Waals contacts** | | | **van der Waals contacts** | | | **van der Waals contacts** | | |
|---|---|---|---|---|---|---|---|---|
| ARG 117 | TYR 966 | | MET 93 | THR 967 | | GLY 689 | THR 750 | |
| LEU 118 | LEU 970 | | | PRO 968 | | THR 750 | GLY 689 | |
| LEU116 | THR 967 | | | LEU 970 | | ILE 795 | GLN 706 | |
| | LEU 970 | | LEU 101 | TYR 739 | | | | |
| | VAL 972 | | PHE 97 | THR 967 | | | | |
| LEU 120 | LEU 970 | | | LEU 970 | | | | |
| | | | LYS 132 | VAL 972 | | | | |
| | | | | MET 973 | | | | |

The following examples are provided to better illustrate particular embodiments, and they should not be considered limiting the application. The application is limited only by the claims.

### Examples

### 1.Structure of ALK/LTK cytokine-binding domains

ALK is an evolutionarily ancient RTK in C. *elegans* and *D. melanogaster,* where it is activated by HEN-1¹⁸ and Jeb^{19,20}, both featuring LDLa domains . The extracellular segment of invertebrate ALK resembles the one in vertebrates but lacks the N-terminal HBD. Whereas invertebrates encode a single ALK family receptor, gene duplication in vertebrates spawned LTK as a second ALK-like receptor²¹. During vertebrate evolution the ALK ectodomain remained constant and divergent evolution of LTK led to loss of the N-terminal HBD plus the MAM-LDLa-MAM cassette in mammals . Yet, the cytokine-binding segment in the ALK and LTK ectodomains bears no resemblance to any known protein-binding domain among cytokine receptors.

To shed light onto the enigmatic structure-function landscape of ALK and LTK, we pursued crystal structures for human ALK and LTK ectodomains comprising their TNFL, GR, and EGFL membrane-proximal segments (Table 1). We produced glycan-shaved ALK_{TG-EGFL} (residues 648-1030), its complex with a non-neutralizing Fab fragment²², and LTK_{TG} (residues 63-380) in mammalian cells ( Fig. 6a,b,c). We obtained crystal structures for ALK_{TG-EGFL} and ALK_{TG-EGFL}-Fab to 3 Å and 2.8 Å resolution, respectively, and for LTK_{TG} to 1.3 Å resolution (Table 1).

Unexpectedly, the TNFL and GR regions in ALK and LTK do not form separate domains, but are intimately interwoven into a large, continuous, and fully globular TG supradomain²³ (Fig. 1b,c, Fig. 6d,e,f). ALK_{TG} and LTK_{TG} display an unprecedented protein fold topology featuring a chimeric arrangement of subdomains with distinct secondary structure as a result of 6 crossover linkages (Fig. 1b-d, Figure 6e,f). The TNFL subdomain is an anti-parallel β-sandwich while the GR subdomain folds into a honeycomb-like lattice of poly-glycine ll-helices (Fig. 1e, Fig. 6g). TNFL and GR interface via an extensive hydrophobic groove lined by conserved residues (Fig. 1f,g). The N-terminus of the TG supradomain maps to the first strand of its TNFL subdomain while its C-terminus is on the adjacent strand (Fig. 1d, Fig. 6d). In our structure of ALK_{TG-EGFL} this connects to the membrane-proximal EGFL module (Cys987 to Pro1025) via a short N-glycosylated linker (Fig. 1b, Fig. 6e). Furthermore, the TG supradomain core is decorated by four peripheral α-helices with α1 tethered to the TNFL subdomain via a conserved disulfide, while the disulfide-linked α2 and α4 cluster at the tip of the GR subdomain together with α3 (Fig. 6e,f).

The GR subdomain displays 14 long and tightly packed pGll-helices arranged in a honeycomb-like lattice (Fig. 1e, Fig. 6g). Analogous pG-II helix networks, albeit much less extensive, have been observed in synthetic polyglycines²⁴ and four functionally diverse proteins spanning all domains of life^{25,26,27,28} (Fig. 6h). Notably, the GR subdomain core has three pGll-helices (d, k, l) that exclusively contain glycine residues. Each of them is surrounded by six other pGll-helices, establishing an interaction network based on compact van der Waals contacts and hydrogen-bonding involving their main-chain amide and carbonyl atoms (Fig. 1e, Fig. 6g). Such tight packing appears to restrict the amino acid composition of these central pGll-helices, offering a rationale for the exquisite conservation of the participating poly-glycine sequences and for loss-of-function mutants in this region of Drosophila ALK²⁹.

### 2.Evolution of ALK/LTK cytokine-binding domains

A query in the DALI server³⁰ using our structural models for ALK_{TNFL} and LTK_{TNFL} retrieved TNF/C1q-class folds (e.g. r.m.s.d =2.8 Å against C1q and TNF, 72 Calpa atoms). However, the ALK_{TNFL}/LTK_{TNFL} chain topology is radically different and unprecedented (Fig. 6i)³¹. Topology-independent searches³² returned more extensive structural superpositions covering an additional ~20 residues in the canonical TNF fold, and structure-based sequence alignments clarified the sequence homology between the A, D and E β-strands in ALK/LTK_{TNFL} and β-strands B, E and F in TNF or TRAIL. The shuffling of spatially equivalent beta-strands between ALK_{TNFL}/LTK_{TNFL} and TNF/C1q proteins goes far beyond a simple permutation (Fig. 6i)³³. The distinctly connected beta-strands in the ALK_{TNFL}/LTK_{TNFL} subdomain break up the alternating sheet-to-sheet register of the TNF/C1q beta-jellyroll, and instead permit the spatially contiguous sprouting of the three glycine-rich loop inserts (between beta-strands D and E, F and G, and H and H') that go on to fold into the distinctive lattice of the ALK_{TNFL}/LTK_{GR} subdomain. There is no simple evolutionary path (by genetic reshuffling) that would lead to this unique, large-scale reconnection of the beta-jellyroll topology from the canonical TNF/C1q structure to the unique ALK_{TNFL}/LTK_{TNFL} fold-that sprouts its remarkable GR subdomain. Such spatial coalescence of three otherwise unremarkable (and conventionally unstructured) stretches of glycine-rich loop insertions to a topologically tortured TNFL domain argues for further study as a new and versatile scaffold for protein design^{34,35}.

### 3.Assembly of ALK/LTK-cytokine complexes

Since ALKAL1 has been reported as an LTK-specific cytokine and ALKAL2 activates both ALK and LTK⁶⁻⁸, we opted to pursue structures of ALK-ALKAL2 and LTK-ALKAL1 complexes. We could readily purify truncated versions of both ALKALs corresponding to the conserved C-terminal domains (termed ALKAL1 and ALKAL2) in HEK293T cells as well as full-length ALKAL1 (ALKAL1_{FL}). All three purified ALKALs were monomeric (Fig. 7a) and could drive ALK-dependent Ba/F3 cell proliferation (Fig. 7b,c). Intriguingly, ALK/LTK-cytokine complexes displayed distinct receptor:cytokine stoichiometries. LTK_{TG-EGFL}-ALKAL1 and LTK_{TG-EGFL}-ALKAL2 complexes were measured as 2:1 stoichiometric assemblies (2 receptors + 1 cytokine) (Fig. 8a,b), whereas ALK_{TG-EGFL}-ALKAL1 and ALK_{TG-EGFL}-ALKAL2 complexes were only compatible with 1:1 stoichiometries (Fig. 8a,c). The same stoichiometric dissonance was observed for the complexes of ALK_{TG} and LTK_{TG} lacking the membrane-proximal EGFL domain (Fig. 8d,e). Such ALK/LTK-cytokine complexes appeared to deviate from the canonical 2:2 stoichiometries among RTK-cytokine complexes^{36,37}, and were rather reminiscent of hematopoietic cytokine-receptor assemblies. After failing to obtain diffraction-quality crystals from purified LTK_{TG-EGFL}-ALKAL1 and ALK_{TG-EGFL}-ALKAL2 complexes, we leveraged ALK/LTK-cytokine complexes lacking the EGFL domain. In the case of LTK, we additionally employed a non-neutralizing single-domain VHH antibody (Nb3.16) fragment. Crystals of ALK_{TG}-ALKAL2 and a tripartite complex comprising LTK_{TG}-ALKAL1-Nb3.16 (Fig. 8f,g) led to structures to 4.2 Å and 1.9 Å, respectively (Fig.2a,b, Table 1).

Our structures revealed that the overarching assembly mode of ALK/LTK-cytokine complexes entails a 2:1 stoichiometric assembly where a single cytokine molecule is cradled proximal to the membrane by two copies of the receptor TG supradomains resulting in receptor dimerization (Fig. 2a,b). The ALK/LTK-cytokine complexes feature three compact interaction interfaces. Sites 1 and 2 correspond to receptor-cytokine interactions and site 3 describes receptor-receptor contacts (Fig. 2c-f). The cytokine ligands are asymmetric three-helix bundles and use helices B and C to contact one copy of their receptors via site 1, and helix A to engage a second copy of the receptor via site 2 (Fig. 2c,f). The dimerized ALK_{TG} and LTK_{TG} supradomains lean against each other at 45° to establish site 3 contacts contributed by the GR subdomains. This creates a surprising receptor assembly with C2-symmetry (Fig. 2a,b,d,e) mediated by cytokines that lack twofold symmetry. Given how the EGFL domain connects to ALK_{TG} (Fig. 1b), we envisage that ALKAL1/2 might be fully encapsulated by cognate full-length receptors over the cell surface.

### 4.Structure of ALKAL1/2 cytokines

ALKAL1 and ALKAL2 adopt highly similar structures (r.m.s.d=0.54 Å, 56 Calfa-atoms) featuring a new type of disulfide-stabilized three-helix bundle, wherein αA connects via a conserved short loop to a helical hairpin constructed from αB and αC, which in turn are tethered by two conserved disulfides (Fig. 3a). The ALKAL1/2 fold is conspicuously open and lacks a classical hydrophobic core as observed in other helical cytokines^{38,39}. Rather, αA only connects to the BC hairpin by inserting lle77 into a hydrophobic pocket formed by Leu117, Tyr110 and the Cys104-Cys113 disulfide (Fig. 3a). Additional stabilization is provided by hydrogen-bonds between Lys73 and the main chain atoms of Cys104 and Tyr98 (Fig. 3a).

Despite their engulfment by the receptor dimers, both ALKAL1 and ALKAL2 display a solvent-exposed hydrophobic cavity defined by conserved residues: those lining the internal BC face (Leu117, Phe94, Tyr98) and the AB loop (Val84, Phe86) and those around the outside rim contributing hydroxyl groups (Fig. 3b,). Such conservation of core residues and cysteine-disulfides suggest application of this fold to all vertebrate ALKALs. The few sequence differences map to two distinct patches contributed by the end of αC and the AB loop, and might provide insights into cytokine specificity. A conserved stretch of ~10 residues preceding αA was not ordered in the reported structures, suggesting they might help to stabilize the soluble forms of these cytokines rather than contribute to direct receptor engagement or possibly help reduce the entropic cost of binding.

### 5.ALK/LTK-cytokine interaction interfaces

Despite being monomeric and lacking symmetry, ALKAL1 and ALKAL2 remarkably dimerize their cognate receptors into highly similar, twofold-symmetric assemblies reminiscent of receptor complexes mediated by erythropoietin and growth hormone^{40,41}. However, these hematopoietic cytokines display a certain degree of pseudo-symmetry through their four-helix bundle. ALKAL1 and ALKAL2 contact the same binding sites on LTK and ALK but display different interaction modalities. In site 1, αB and αC use a hydrophobic epicenter surrounded by polar residues to contact the TNFL subdomain via a patch contributed by its D, E, H" and I strands and the H"-I loop. In each complex, a trio of arginine residues in ALKAL1 (Arg102, Arg112, Arg119) and ALKAL2 (Arg123, Arg133, Arg140) engages two conserved glutamate residues at the periphery of site 1 (Fig. 4a,b,Fig. 9a, Table 2). These electrostatic interactions are supplemented by Arg115 of ALKAL1 (Arg136 in ALKAL2) running perpendicular to the crossover H"-I loop of the TNFL subdomain and hydrogen-bonding to the main chain carbonyl group of a conserved valine (Fig. 4a,b). On the ligand side, site 1 encapsulates a hydrophobic core contributed by three conserved leucine residues in ALKAL1 and ALKAL2. Here, LTK offers a broader platform by presenting four conserved hydrophobic residues (Phe143, Leu361, Leu364, Val366) (Fig. 4b). Interestingly, the corresponding pocket on ALK substitutes for Phe143 and Leu361 via Ser758 and Thr967, respectively (Fig. 4a, Fig. 9b).

In site 2, which is predominantly hydrophobic, the short ALKAL αA pairs with the tip of the BC hairpin to engage the second receptor molecule (Fig. 4c,d). In the LTK_{TG}-ALKAL1 complex Phe80 protrudes from αA in ALKAL1 into a conserved hydrophobic pocket on LTK (Val366, Tyr124, Phe143). The neighboring Phe76 inserts between Leu361 and Leu364 at the edge of the site 2 interface. Surprisingly, the ALK_{TG}-ALKAL2 complex is devoid of an equivalent residue for Phe143 (Fig. 9c).

Cytokine-mediated dimerization of the ALK_{TG} and LTK_{TG} supradomains induces receptor-receptor contacts (site 3) by locking α1 of the TNFL subdomain with α2 and α3 at the distal end of the GR subdomain (Fig. 2d,e, Fig. 9d). However, the two site 3 interfaces in LTK and ALK are distinct, with the latter being less extensive (Fig. 4e,f, Fig. 9d-f). In LTK, site 3 entails an interaction platform centered on His153 stacking against the peptide of Gly74, analogous to key interactions in IL-6/IL-12 family complexes⁴² (Fig. 4e). Additional interactions are mediated by Arg241 on an LTK-specific loop insert (Fig. 4e, Fig. 9e). At the center of site 3, Asn369 hydrogen-bonds with its symmetry related counterpart (Fig. 9f).

### 6.Site 1 drives ALK/LTK-cytokine complexes

To obtain insights into the contribution of the two distinct ALK/LTK-cytokine interfaces, we used mutants probing the polar interactions of site 1 and the central hydrophobic pocket in site 2. This is especially important to clarify for ALK since its extracellular domain, in contrast to LTK, does not readily proceed to 2:1 stoichiometric complexes with cytokines at the concentrations attainable in solution.

Site 1 contacts were interrogated by introducing charge-reversal mutations of two conserved polar interactions resulting in ALKAL1 mutants ALKAL1^{R102E}, ALKAL1^{R115E}, and ALKALI^{R102E/R115E}, and ALKAL2 mutants ALKAL2^{R123E}, ALKAL2^{R136E} and ALKAL2^{R123E/R136E}. We first established that ALKAL2 is the high-affinity cytokine for ALK and ALKAL1 for LTK and using these binding benchmarks concluded that all site 1 mutants for ALKAL1 and ALKAL2 drastically reduced their affinity to both receptors (Fig. 10a,b).

To probe site 2 we used mutants ALKAL1^{F76E} and ALKAL2^{F97E} aimed at their respective hydrophobic interaction patches, and a ALKAL2^{H100A} mutant. The interaction of ALKAL1^{176E}, ALKAL2^{F97E} and ALKAL2^{H100A} with LTK resulted in a biphasic binding profile with one interaction obeying faster off-rates (Fig. 10c,d). On the other hand, the binding profile of ALKAL2^{F97E} to ALK was similar to wildtype ALKAL2 (Fig. 10e).

We used purified ALKAL1/2 mutants to further investigate the role of sites 1 and 2 via our Ba/F3 cellular proliferation assay (Extended Data Fig. |7f). Whereas wildtype ALKALs induced clear cell proliferation at 10 nM, site 1 double mutants did not (Fig. 4g,h, Fig. 10g). In contrast to our kinetic binding assays, ALKAL2^{F97E} appeared to abrogate the site 2 interaction showing cell proliferation similar to the functionally deleterious site 1 mutant ALKAL2^{R123E/R136E} (Fig. 4g, Fig. 10g). Moreover, we found that site 2 mutants retained their ability to form 1:1 stoichiometric complexes with both ALK and LTK, whereas site 1 mutants did not (Fig. 10h).

Finally, we interrogated the functional importance of site 3 in LTK and ALK. For LTK, mutants LTK^{R241A} and LTK^{R241A/N369G} had a decreased propensity to form the native 2:1 stoichiometric complexes with ALKAL1 (Fig. 10i). Given the resistance of ALK ectodomains to establish 2:1 stoichiometric complexes with cytokines at concentrations applicable in chromatographic methods, we established a Ba/F3 cell line expressing ALK^{M751T} hypothesizing that this mutation would introduce an N-linked glycosylation site at Asn749. We found that ALK^{M751T} was unable to drive cytokine-dependent cellular proliferation while it expresses very similarly to wildtype ALK (Fig. 10j).

The presented structural landscapes of ALK/LTK-cytokine complexes revealed key differences in site 2 and site 3, prompting the question whether evolutionarily conserved determinants might be at play. Indeed, we noted the absence of equivalents for Phe143 and His135 in ALK differentiates ALK from LTK across vertebrates. Together with an insert in the LTK e-f loop (Extended Data Fig. 8a), these interactions create an LTK-specific zipper across the length of the site 3 interface acting as driving force for the apparent increased propensity to form a ternary complex (Fig. 9e,f).

Collectively, our structure-function data suggest that site 1 engagement is the driving force for establishing ALK/LTK-cytokine encounter complexes. Given the high sequence conservation of interfacing residues in both cytokines and receptors the observed ALKAL1/2 binding modes will likely apply to all vertebrate receptors consistent with the reciprocal species cross-reactivity of zebrafish ALKAL2a and human ALKALs⁴³. Interestingly, surface amino acids on the opposite side of the ALK cytokine-binding interfaces are highly conserved, in contrast to LTK, suggesting that they might be relevant for interactions with the N-terminal domains, which are absent in LTK.

### 7.Mapping of somatic mutations in ALK

To expand structure-function insights on ALK and LTK activation we structurally mapped mutations in ALK_{TG} and LTK_{TG} combining documented oncogenic potential and frequently occurring missense single-nucleotide polymorphisms^{44,45} (Fig. 11a,b). Mutations leading to constitutive receptor activation or that may enhance receptor-receptor contacts or stabilize active receptor states are widely studied to evaluate oncogenic potential. LTK variant R243Q and ALK variants G685R, G747R, and H694R locate at positions that would be compatible with such roles. Interestingly, H694R is a gain-of-function mutation in lung adenocarcinoma leading to constitutive activation of ALK⁴⁶. On the other hand, mutations that may impact cytokine binding, e.g. by increasing affinity, are less common. Here, we identify two such candidate mutations: LTK variant P363L and ALK variant S737L mapping to interaction sites 2 and 1, respectively.

ALK^{F856S}, a gain-of-function mutation linked to acute myeloid leukemia⁴⁷, and ALK^{R753Q} identified in histiocytic neoplasms⁴⁸, are roughly equidistant from the start of αC of bound ALKAL2 (Fig. 11a). To gain insight into their possible mechanism, we established Ba/F3 cell lines expressing the two mutant ALK variants and found that while their expression levels were comparable to ALK^{WT}, they both drastically increased cytokine-dependent cell proliferation (Fig. 11c,d,e,f). Unlike previously reported⁴⁷, ALK^{F856S} was not constitutively active. In light of their structural context, these mutations might facilitate the reorganization of key regions of the cytokine-receptor interfaces.

### 8.Cytokine specificity in ALK by the EGFL domain

Given the overall structural similarity of ALK_{TG}/LTK_{TG}-cytokine complexes and that EGFL modules can bind ligands⁴⁹, we hypothesized that the membrane-proximal EGFL domain might underlie the apparent preference of ALK for ALKAL2 over ALKAL1. Benchmarking of the binding thermodynamics showed that even at micromolar concentrations, ALK_{TG-EGFL} formed enthalpically-driven binary complexes with either cytokine characterized by a markedly higher affinity for ALKAL2 (*K_{D}* = 40 nM) than ALKAL1 (*K_{D}* = 600 nM) (Fig. 5a-c). In contrast, LTK_{TG-EGFL} bound with high-affinity and 2:1 stoichiometry to both cytokines (*K_{D}*, _{ALKAL1} = 10 nM, *K*_{D}, _{ALKAL2} = 55 nM) (Fig. 12a,b) in agreement with stoichiometries derived from small-angle X-ray scattering analyses (Fig. 12c). As we could produce ALKAL1 carrying the N-terminal region absent in our structure, we titrated LTK_{TG-EGFL} with ALKAL1_{FL} and measured a modest increase in affinity compared to the truncated form (Fig. 12d).

Sequence differences in ALKAL1 and ALKAL2 map to distinct patches proximal to the EGFL domain (Fig. 12e). Remarkably, removal of the EGFL domain from ALK resulted in a drastic 30-fold decrease in affinity for ALKAL2 but only a 4-fold affinity reduction for ALKAL1 (Fig. 5d,e). In contrast, the LTK_{TG} domain retained its nanomolar binding affinity to both ALKAL1 and ALKAL2 (Fig. 12f-h). Thus, while ALK_{TG} is endowed with a baseline micromolar affinity for both cytokines, the receptor's specificity for ALKAL2 is substantially enhanced by the EGFL domain.

As ALK carries four additional N-terminal domains compared to LTK (Fig. 1a), we measured the binding affinity of full-length ALK ectodomain (ALK_{FL}) for ALKAL2. We obtained a similar affinity and 1:1 stoichiometry to the ALK_{TG-EGFL}-ALKAL2 interaction indicating that the N-terminal domains of ALK play a negligible role in cytokine binding (Fig. 12i). Nevertheless, in line with the reported interaction of canine ALK with heparin⁹, we found that heparin induced dimerization of a large fraction of human ALK_{FL} in the presence or absence of bound cytokine (Fig. 12j). In hindsight, the inordinately high protein concentration in protein crystals compared to what can be attained in solution appears to have fortuitously compensated for the deficiency of ALK_{TG} to undergo cytokine-induced dimerization in solution.

### 9.Mechanistic considerations

Whereas cytokine-mediated dimerization of ALK and LTK leads to structurally similar ternary complexes, the mechanistic requirements for their assembly appear to be distinct. LTK-cytokine complexes are fully cytokine-driven whereas ALK-cytokine complexes might synergize with glycosaminoglycans (Fig. 5f). Our reported ALK/LTK-cytokine complexes are likely representative in all vertebrates because of the strong sequence conservation in receptors and ligands, and their species cross-reactivity^{6,43}. However, invertebrate ALK ligands are unable to activate human ALK and are structurally distinct from ALKAL1/2⁵⁰. The currently known modes of ligand-induced activation of RTKs display a broad array of structural principles^{36,37}, ranging from dimerized receptor assemblies exclusively driven by the activating ligands (e.g. Trka-NGF)⁵¹ to complexes mediated fully via receptor contacts (e.g. EGFR-EGF)⁵². However, several cytokine-RTK assemblies feature an amalgamation of ligand- and receptor-mediated contributions (e.g. CSF-1/IL-34-CSF-1R)^{53,54} including the involvement of accessory molecules (e.g. FGF-FGFR)⁵⁵ or co-receptors^{56,57}, and the use of multiple cytokine copies to (e.g. EGFR and Insulin receptor)^{52,58}. The utilization of a single copy of a monomeric cytokine by ALK family receptors to undergo dimerization with twofold symmetry introduces a novel cytokine-driven assembly mechanism among RTK (Fig. 12k).

It is now clear that key differences in the cytokine-binding regions of ALK and LTK dictate cytokine specificity and that receptor-receptor contacts are also important differentiating factors. The resistance of isolated ALK ectodomains towards cytokine-induced dimerization suggests that the reduced dimensionality of their membrane-proximal engagement and additional interactions of its N-terminal domains with glycosaminoglycans or proteoglycans⁸ might be important for productive cytokine-receptor assemblies, much like bound heparin bridging receptors in FGF-FGFR complexes. In this context, the reported proteolytic shedding of the N-terminal segment of ALK's ectodomain presents with a physiological conudrum⁵⁹. Intriguingly, the EGFL module of ALK, but not LTK, appears to dictate cytokine specificity, such that the mode of its engagement in ALK-cytokine complexes may impact the orientation of the membrane-proximal domains (and their connected transmembrane helices) to fine tune signaling assemblies.

We envisage application of our findings to further interrogate ALK/LTK signaling in physiology and disease, and in the therapeutic targeting of the ALK/LTK ectodomains¹⁷ and their cognate cytokines¹⁶.

### Materials and methods

No statistical methods were used to predetermine sample size. The experiments were not randomized and the investigators were not blinded to allocation during experiments and outcome assessment.

### 1.Plasmids, constructs, and cell lines for protein expression in mammalian cells

Sequence optimized DNA for full length wild-type ALK (Uniprot ID Q9UM73), LTK (Uniprot ID P29376), ALKAL1 (Uniprot ID Q6UXT8) and ALKAL2 (Uniprot ID Q6UX46) were purchased from Genscript. DNA encoding for different human ALK constructs comprising either amino acids 19-1025 (ALK_{FL}), 648-1025 (ALK_{TG-EGFL}) or 648-985 (ALK_{TG}) and human LTK constructs comprising amino acids 63-420 (LTK_{TG-EGFL}) or 63- 379 (LTK_{TG}) were cloned in the pHLsec vector⁶⁰ in frame with a N-terminal chicken RTPµ-like signal peptide sequence and a C-terminal caspase3 cleavable Fc-Hisₓ₆-tag at the C-terminus.

Sequences encoding for ALKAL1_{FL} (residues 28-129), ALKAL1 (residues 57-129) and ALKAL2 (residues 78-152) were cloned in the pHLsec vector in frame with a N-terminal chicken RTPµ-like signal peptide sequence followed by a caspase3 cleavable Sumostar-tag at the C-terminus. Sequence-optimized DNA encoding the light and heavy chains of Fab324 were purchased from IDT as GBlocks. The N-terminal signal peptide sequences were exchanged for a chicken RTPµ-like signal peptide sequence. The heavy chain was cloned in frame with a C-terminal caspase-3 site followed by an AVI-His₆ₓ tag, while the light chain was cloned without a purification tag.

### 2.Protein expression in HEK293 and purification from conditioned media

Production of all ALK_{TG-EGFL} and ALK_{TG} constructs was performed in adherently grown HEK293 *MGAT1*^{*-*/*-*} cells⁶¹ maintained in DMEM supplemented with 10% FCS. When cells reached 80% confluency they were transiently transfected using branched polyethylenimine 25kDa as transfection reagent in DMEM with 3.6 mM valproic acid and without FCS.

Expression of the Fab fragment was achieved in adherent HEK293T cells using the same method. For the heterodimeric Fab fragment, plasmids encoding for each chain were co-transfected in a 1:1 ratio. Protein production for ALK_{FL}, ALKAL1/2 and LTK constructs was performed in HEK293S cells grown in suspension and maintained in a mixture of 50% Freestyle (Thermofisher) / 50% Ex-Cell (Sigma-Aldrich) growth medium. Transient transfection was performed with linear polyethylenimine (Polysciences) 25kDa as transfection reagent. One day after transfection valproic acid was added until a final concentration of 1.5 mM⁶².

For expression in suspension cells conditioned medium was harvested after five days and subsequently clarified by centrifugation for 12 minutes at 8000 xg while medium from adherently grown cells was harvested after 6 days and centrifuged for 15 minutes at 6000 xg. After centrifugation media were filtered through a 0.22 mm filter prior to chromatographic purification steps.

ALK and LTK constructs were captured via their Fc-tag on a protein A column (HiTrap Protein A HP, Cytiva) and eluted via on-column digest with caspase 3 for 1 hour at 37°C followed by 2 hours at room temperature and eluted with HBS (HBS (20mM HEPES pH 7.4 150mM NaCl). The eluted proteins were then concentrated and injected on a HiLoad 16/600 SD200 (Cytiva) size-exclusion chromatography column pre-equilibrated with HBS. Purified proteins were stored at -80°C until further use.

ALKAL containing medium was fourfold diluted with 20mM HEPES pH 7.4 before loading on a cation exchange column packed with SP Sepharose Fast Flow resin (Cytiva) equilibrated in 20mM HEPES pH7.4 50mM NaCl. ALKALs were eluted using a NaCl gradient from 50mM-750mM for 20 minutes. Fractions containing ALKALs were immediately diluted with 20mM HEPES pH7.4 to a NaCl concentration of 200 mM and further supplemented with 0.1% (w/v) CHAPS. The C-terminal Sumo-tag was cleaved with caspase 3 overnight at 20°C. To remove undigested protein as well as the cleaved Sumo-tag, the digestion mixture was loaded on a MonoQ 5/50 GL column (Cytiva). Flowthrough containing the ALKALs was concentrated and injected on a Superdex 75 Increase 10/300 GL equilibrated in HBS supplemented with 0.1% CHAPS. Purified ALKALs were stored at -80°C at a concentration of 1mg ml⁻¹ until further use. For ALKALs used in BaF/3 assays, endotoxin levels were measured using a Endosafe PTS limulus amebocyte lysate assay (Charles river) and were below 5 EU mg⁻¹.

Fab fragments were captured using cOmplete His-tag purification resin (Roche) and eluted using HBS supplemented with 250 mM imidazole followed by buffer exchange to HBS on a HIPrep 26/10 desalting column. Caspase 3 was added to the purified Fab fragment in order to remove the AviHis tag of the heavy chain by overnight digestion at 20°C. The sample was loaded on an IMAC column in order to remove the enzyme and undigested protein. The flow-through containing tagless Fab fragments was concentrated and injected on a Superdex 200 Increase 10/300 GL (Cytiva) column pre-equilibrated in HBS.

### 3.Production of non-neutralizing single domain camelid VHH against LTK

Single domain camelid VHHs (Nanobodies) against LTK were raised by immunizing llamas with LTK_{TG-EGFL} and were selected for specific binding to LTK_{TG-EGFL} via ELISA and BLI. Epitope binning via BLI led to the identification of candidate Nanobodies with non-neutralizing behavior with respect to cytokine binding. The sequences of such non-neutralizing Nanobodies were cloned in a MoClo derived yeast expression vector in frame with a N-terminal preproMF secretory leader sequence followed by the N-terminal Hisₓ₆ tag and a caspase cleavage site. *Komagataella phaffii* cells were transformed by electroporation and grown on YPDS agar containing 500 µg/ml zeocin. One colony was picked to inoculate 500 ml of BMGY supplemented with 100 µg/ml zeocin and grown at 28°C for 24 hours. Next, cells were pelleted by centrifugation at 500xg for 7 minutes and resuspended in 500ml BMMY medium without zeocin and incubated O/N at 28°C. Expression was further induced by adding 2.5 ml of 50% methanol, the same volume of methanol was again added after 8h and 24h. After which cells were incubated for another 8h at 28°C. Finally, conditioned medium was harvested by centrifugation for 10 minutes at 6000xg.

His-tagged camelid single domain VHHs were captured by addition of 2ml cOmplete resin (Roche) to 500 ml conditioned medium followed by overnight incubation at 4°C while shaking. Nanobodies were eluted in HBS supplemented with 250mM imidazole and buffer exchanged to HBS on a HiPrep 26/10 desalting column (Cytiva). The N-terminal Hisₓ₆ tag was removed by an overnight caspase3 digest at 20°C. Undigested protein and the enzyme were removed via IMAC. The flowthrough containing tagless nanobody was concentrated and injected on an SD75 Increase 10/300 GL column (Cytiva) pre-equilibrated in HBS. Purified nanobody was concentrated to a concentration of 4 mg ml⁻¹ and flash-frozen and stored at - 80 °C.

### 4.Crystallization and crystal structure determination.

For ALK_{TG-EGFL}_Fab324 crystals, a 1.5 molar excess of Fab324 was added to ALK_{TG-EGFL} and further subjected to an over-night enzymatic digestion of N-linked glycans with EndoH. The complex was polished by SEC on a Superdex 200 Increase 10/300 GL (Cytiva) and concentrated to 13.5 mg ml⁻¹. Commercial sparse matrix crystallization screens were set up using a Mosquito liquid handling robot (TTP Labtech) in sitting drop format using 100 nL protein mixed with 100 nL mother liquor in SwissSci 96-well triple drop plates incubated at 287 K. A first hit in the Morpheus II screen⁶³ was further optimized to a condition consisting of (40 mM Polyamines, 0.1 M Gly-Gly/AMPD pH 8.5, 11% w/v PEG4000, 19 % w/v 1,2,6-Hexanetriol) in sitting drop format with a 100 nl protein mixed with 200 nL mother liquor geometry. Crystals were cryoprotected in mother liquor containing 25% w/v 1,2,6-Hexanetriol prior to being cryocooled in liquid nitrogen. Diffraction data was collected at 100 K at the ID23-2 beam line at ESRF, Grenoble. The datasets were processed using XDS⁶⁴. Initial phases were calculated by molecular replacement with PHASER⁶⁵ using the coordinates of a Fab fragment exhibiting the highest sequence identity (PDB: 5nuz, chain A ) followed by rigid body refinement in Buster⁶⁶. A partial polyalanine model was built into the visible electron density in Coot⁶⁷ followed by density modification via Resolve⁶⁸. The density modified map showed density for several aromatic sidechains allowing for assignment of the correct register and tracing of the ALK sequence. Additional refinement steps were carried out in PHENIX⁶⁹ using individual B-factor refinement in combination with TLS, XYZ refinement, optimizing the X-ray/geometry weights as well as local torsion angle non-crystallographic symmetry (NCS) restraints.

For crystals of ALK_{TG-EGF}, glycans were trimmed by over-night enzymatic digestion with EndoH in HBS. The complex was polished via SEC and concentrated to 10.5 mg ml⁻¹. Commercial sparse matrix sitting drop crystallization screens were set up as described. One hit was obtained in the Morpheus II screen and optimized to 0.5mM Manganese(II) chloride tetrahydrate, 0.5mM Cobalt(II) chloride hexahydrate, 0.5mM Nickel(II) chloride hexahydrate, 0.5mM Zinc acetate dihydrate, 13% w/v PEG 3000, 28% v/v 1,2,4-Butanetriol, 1% w/v NDSB 256 in hanging drop format. Crystals were cryoprotected in mother liquor containing 25% 1,2,4-Butanetriol prior to being flash frozen in liquid nitrogen. Diffraction data was collected at 100 K at the P14 microfocus beam line at PETRA III, Hamburg and integrated using XDS with standard parameters except for the "BEAM_DIVERGENCE" parameter which was doubled. Initial phases were obtained using maximum likelihood molecular replacement in Phaser using the structure of the ALK_{TG} domain. The structure was refined using Phenix.refine followed by manual building in COOT. The EGF-like domain was manually built into the electron density. Refinement in phenix followed the same protocol as for the ALK_{TG-EGFL}_Fab324 structure except for the absence of NCS restraints and implementation of additional reference restraints based on the structure of ALK_{TG} in complex with Fab324.

For crystals of LTK_{TG}, purified LTK_{TG} was concentrated to 10 mg ml⁻¹ and used to set up sparse matrix screens as previously described. Crystals appeared in a condition of the Morpheus II screen with the final optimized condition containing (MOPSO/Bis-Tris pH 6.3, 12% PEG 20000, 26% Trimethyl propane 1% w/v NDSB 195 5mM Yttrium (III) chloride hexahydrate, 5mM Erbium (III) chloride hexahydrate, 5mM Terbium(III) chloride hexahydrate, 5mM Ytterbium (III) chloride hexahydrate) set-up in sitting drop format with a 100 nL protein mixed with 200 nL mother liquor geometry was cryoprotected in mother liquor and cryo-cooled in liquid nitrogen. Diffraction data was collected at 100K at the P13 microfocus beam line at PETRAIII, Hamburg and processed using XDS as previously described. Phases were obtained by single wavelength anomalous dispersion making use of the anomalous signal from lanthanide atoms. Determination of the lanthanide substructure for four sites was performed by the hybrid substructure search as implemented in Phenix. Phases were calculated using Phaser-EP. The density was readily interpretable, and a model was manually built in Coot and further refined in Phenix implementing an anisotropic individual B-factor model.

For LTK_{TG}-ALKAL1-Nb3.16 crystals a 3-fold molar excess of Nb3.16 was added to the LTK_{TG}-ALKAL1 complex, concentrated and injected into a Superdex 200 Increase 10/300 GL (Cytiva) equilibrated in HBS. Eluted fractions were concentrated to 13.5 mg ml⁻¹ and used to set up sitting drop crystallization screens as described. Crystals were obtained in a condition consisting of MOPSO/BIS-TRIS pH 6.3 13% PEG 8000, 22% w/v 1,5-pentanediol, 5mM sodium chromate tetrahydrate, 5mM sodium molybdate tetrahydrate, 5mM sodium tungstate tetrahydrate, 5mM sodium orthovanadate tetrahydrate. Crystals were cryoprotected in mother liquor containing 25% 1,2,4-Butanetriol prior to being flash frozen in liquid nitrogen. Diffraction experiments were performed at 100 K Proxima 2 microfocus beam line at the Soleil synchrotron. Initial phases were obtained by maximum likelihood molecular replacement using Phaser with the previously obtained LTK_{TG} structure. A model for Nb3.16 was automatically built using ArpWarp^{70,71} followed by manual building of ALKAL1 in Coot. Refinement was performed in Phenix with individual anisotropic ADP parameters with a TLS model.

For ALK_{TG}-ALKAL2 crystals a 3-fold molar excess of ALKAL2 was added to ALK_{TG} and subjected to an overnight EndoH digest, concentrated and injected into a Superdex 200 Increase 10/300 GL (Cytiva) equilibrated in HBS. Eluted fractions were concentrated to 8 mg ml⁻¹ and used to set up sitting drop crystallization screens as described. Initial hits were obtained in a condition consisting of 0.1M MES pH 6.5 15% w/v PEG 6000 5% w/v MPD. A single crystal was used to prepare a seed stock⁷² using the PTFE seed bead (Hampton Research). The best diffracting crystals were obtained by seeding with a 1:1000 dilution of the seed stock into the optimization screen. Crystals were cryoprotected in 78% (v/v) mother-liquor supplemented with 22% (v/v) ethylene glycol before flash freezing in liquid nitrogen. Diffraction data were collected at 100 K at the Proxima 2 microfocus beam line at the Soleil synchrotron. Data as processed as described above with the difference that anisotropy correction was implemented by the UCLA diffraction anisotropy server⁷³. Initial phases were obtained by molecular replacement in Phaser using the previously determined ALK_{TG} and ALKAL1 structures. First refinement cycles were performed in Buster followed by iterative refinement using Coot and Phenix. B-factors were refined using two isotropic atomic displacement parameters complemented by TLS. During refinement structures of ALK_{TG} and ALKAL1 provided reference model restraints.

For Fab324 crystals, tag-free Fab324 was concentrated to 18.5 mg ml⁻¹ and a pH versus (NH₄)₂SO₄ concentration screen was set up in sitting drop format resulting in crystals in a condition consisting of (1.5M (NH₄)₂SO₄ 40 mM Glycine pH 9.5). The crystal was cryoprotected using a saturated (NH₄)₂SO₄ solution. Diffraction experiments were performed at the P13 beamline Petralll, Hamburg and data were processed using XDS. Phases were obtained by molecular replacement using our structure of Fab324 obtained from the ALK_{TG-EGFL}_Fab324 complex. The structure was refined using Coot and Phenix.

All display items containing structures were generated using the PyMOL Molecular Graphics System, version 2.0.5 (Schrödinger).

### 5.lsothermal Titration Calorimetry (ITC)

Experiments were performed using a MicroCal PEAQ-ITC instrument at 310K. Proteins used in ITC experiments were expressed in HEK293S cells grown in suspension. As a final purification step all proteins were buffer exchanged to the same HBS buffer via size-exclusion chromatography. Titrations were preceded by an initial injection of 0.5 µL. The injection spacing was optimized per experiment to allow for the signal to get back to a stable baseline. Throughout the titration the sample was stirred at a speed of 750 r.p.m. Data were analyzed using the PEAQ-ITC analysis software (version 1.1.0.1262, Malvern) and fit using a "one set of sites" model.

### 6.Multi-angle Laser Light Scattering (MALLS)

Protein samples of 100 µl at approximately 1.0 mg ml⁻¹ were injected onto a Superdex 200 increase 10/300 GL column (Cytiva) connected in line to a UV-detector (Shimadzu), a miniDawnTReos (Wyatt) multi-angle laser light scattering detector and an optilab T-Rex (Wyatt) refractometer. The refractive increment value (dn/dc) was 0.185 mL g⁻¹. Band broadening was corrected for using reference measurements of BSA (Pierce). Data analysis was carried out using the Astra 6.1.6 software and standard deviations were calculated using Prism8 (Graphpad Software).

### 7.Biolayer interferometry (BLI)

Screening of mutant ALKALs was performed by immobilizing wild-type and mutant ALKAL1/2 variants. To this end, residues 57-129 of wild-type ALKAL1 and interface mutants (R102E, R115E, R102E/R115E, F76E and F80E) as well as residues 78-152 of wild-type ALKAL2 and interface mutants (R123E, R136E, R123E/R136E, F97E and H100A) were cloned in the pHLsec vector in frame with a C-terminal Avi tag. All constructs were transiently cotransfected in suspension grown HEK293S cells together with a BirA expression plasmid (pDisplayBirA-ER⁷⁴) as previously described and supplemented with 100 µM biotin upon transfection. After 4 days of expression, excess biotin was removed by desalting the conditioned media to HBS on a HIPrep 26/10 desalting column (Cytiva).

All measurements of binding kinetics and dissociation constants were performed using an Octet Red 96 (Forté Bio) in assay buffer (20 mM HEPES pH 7.4, 150 mM NaCl, 0.02% (w/v) BSA, 0.002% (v/v) Tween 20) at 298K. ALKALs were immobilized to a level of 0.5 nm on streptavidin-coated biosensors (Forté Bio). To verify that no aspecific binding was present during the assay, non-functionalized biosensors were used as a control by measuring in parallel all ligand concentrations as well as running buffer. For all mutants a two-fold dilution series from 6.4 µM-400 nM was employed. Data analysis was performed using the Data Analysis software 9.0.0.14 (Forté Bio) and binding curves were exported to Prism8 (Graphpad Software) for plotting of curves.

### 8.Small angle X-ray scattering (SAXS)

SEC-SAXS data were collected at the SWING beamline at SOLEIL (France) using an integrated online HPLC set-up. Purified samples of ALK_{TG-EGFL}-ALKAL2 (18.5 mg ml⁻¹) and LTK_{TG-EGFL}-ALKAL1 (19 mg ml⁻¹) expressed in HEK293SMGAT^{-/-} cells were injected on a Biosec-3 column (Agilent) with HBS as a running buffer. The scattering data were collected in continuous flow mode with a flow speed of 0.3ml/min and a 1 s exposure time per frame. Buffer and sample frames were selected and subtracted using the program RAW⁷⁵. Theoretical scattering curves and fitting to experimental scattering data was performed with AllosMod-FoXS.

Briefly, a model for the C-terminal EGFL domain of LTK was generated by homology modeling starting from the crystal structure of the ALK EGF-like domain using the SWISS-MODEL server⁷⁶. This model was manually placed and connected to the C-terminus of the LTK_{TG} domain in Pymol based on the ALK_{TG-EGFL} structure. Missing regions in the ALK and LTK structures were added using MODELER⁷⁷. The resulting models were subsequently energy minimized using Rosetta-Relax, and used as an input for AllosMod to add N-linked glycans on positions Asn709, Asn808, Asn886 and Asn986 for ALK and Asn380 and Asn412 for LTK, and calculated resulting model energy landscapes. The output of AllosMod was then used in AllosMOD-FoXS to calculate fits with theoretical scattering curves during fast AllosMod simulations at 300 K.

### 9.Cell culture and retroviral transduction

Ba/F3 cells (murine pro-B cell line) cells were cultured in RPMI/10% FCS supplemented with mouse interleukin-3 (IL-3, 1 ng ml⁻¹). Ba/F3 cell line was not listed in the database of commonly misidentified cell lines maintained by ICLAC and NCBI Biosample. Ba/F3 cells were transduced with viral supernatant MSCV-ALK^{WT}/ALK^{R753Q}/ ALK^{F856S}EV-IRES-GFP (EV; empty vector) for 2 days in RPMI/10% FCS supplemented with mouse + IL-3 as previously described⁷⁸. GFP-sorted cells were used for the cell growth assays and western blot. After removal of IL-3 from the media, the cells were stringently washed with PBS for three times. The cell growth curves and heatmaps were made using GraphPad Prism 9 software as mean values, with error bars representing standard deviation.

### 10. Reagents

For Western blotting, the following antibodies were used: ALK (Purchased from Cell Signaling Technologies; catalog no.: 3633; dilution: 1:1,000), Phospho-ALK (Tyr1278) (Cell Signaling Technologies; 6941; 1:250), Phospho-ALK (Tyr1604) (Cell Signaling Technologies; 3341; 1:250), Phospho-p44/42 MAPK (Erk1/2) (Thr202/Tyr204) (Cell Signaling Technologies; 4370; 1:2,000), p44/42 MAPK(Erk1/2) (Cell Signaling Technologies; 9102; 1:1,000), β-actin (Sigma-Aldrich; A-5441; 1:2,000), GAPDH (GENETEX; GTX100118; 1:2000). For the cell growth assay, Crizotinib (Sigma-Aldrich; PZ0191-5MG), DMSO (Signa-Aldrich; D8418-100ML), and IL-3 (Peprotech; AF-213-13) were used.

### 11.Quantification and statistical analysis

### Statistics and reproducibility Ba/F3 assay

Statistical significance was determined by two-way ANOVA followed by Tukey's multiple comparison test where multiple comparisons should be adjusted. Data were plotted using GraphPad Prism 9 software as mean values, with error bars representing standard deviation. Heatmaps were also made using GraphPad Prism 9 software based on mean values. *P < 0.05, ** P < 0.01 and *** P < 0.001, respectivNb3.16ely, unless otherwise specified.

### References

1. Ben-Neriah, Y. & Bauskin, A. R. Leukocytes express a novel gene encoding a putative transmembrane protein-kinase devoid of an extracellular domain. Nature 333, 672-676 (1988).
2. Morris, S. W. et al. Fusion of a kinase gene, ALK, to a nucleolar protein gene, NPM, in non-Hodgkin's lymphoma. Science (80-. ). 263, 1281-1284 (1994).
3. Orthofer, M. et al. Identification of ALK in Thinness. Cell 181, 1246-1262.e22 (2020).
4. Blum, M. et al. The InterPro protein families and domains database: 20 years on. Nucleic Acids Res. 49, D344-D354 (2021).
5. Reshetnyak, A. V. et al. Identification of a biologically active fragment of ALK and LTK-Ligand 2 (augmentor-α). Proc. Natl. Acad. Sci. U. S. A. 115, 8340-8345 (2018).
6. Zhang, H. et al. Deorphanization of the human leukocyte tyrosine kinase (LTK) receptor by a signaling screen of the extracellular proteome. Proc. Natl. Acad. Sci. U. S. A. 111, 15741-15745 (2014).
7. Reshetnyak, A. V. et al. Augmentor α and β (FAM150) are ligands of the receptor tyrosine kinases ALK and LTK: Hierarchy and specificity of ligand-receptor interactions. Proc. Natl. Acad. Sci. U. S. A. 112, 15862-15867 (2015).
8. Guan, J. et al. FAM150A and FAM150B are activating ligands for anaplastic lymphoma kinase. Elife 4, (2015).
9. Murray, P. B. et al. Heparin is an activating ligand of the orphan receptor tyrosine kinase ALK. Sci. Signal. 8, ra6-ra6 (2015).
10. Hallberg, B. & Palmer, R. H. Mechanistic insight into ALK receptor tyrosine kinase in human cancer biology. Nature Reviews Cancer vol. 13 685-700 (2013).
11. Janostiak, R., Malvi, P. & Wajapeyee, N. Anaplastic Lymphoma Kinase Confers Resistance to BRAF Kinase Inhibitors in Melanoma. iScience 16, 453-467 (2019).
12. Javanmardi, N. et al. Analysis of ALK , MYCN , and the ALK ligand ALKAL2 ( FAM150B/AUGα ) in neuroblastoma patient samples with chromosome arm 2p rearrangements. Genes, Chromosom. Cancer 59, 50-57 (2020).
13. Li, N. et al. Gain-of-function polymorphism in mouse and human Ltk: Implications for the pathogenesis of systemic lupus erythematosus. Hum. Mol. Genet. 13, 171-179 (2004).
14. Müller-Tidow, C. et al. High-Throughput Analysis of Genome-Wide Receptor Tyrosine Kinase Expression in Human Cancers Identifies Potential Novel Drug Targets. Clin. Cancer Res. 10, 1241-1249 (2004).
15. Pospisilik, J. A. et al. Drosophila Genome-wide Obesity Screen Reveals Hedgehog as a Determinant of Brown versus White Adipose Cell Fate. Cell 140, 148-160 (2010).
16. Borenäs, M. et al. ALK ligand ALKAL2 potentiates MYCN-driven neuroblastoma in the absence of ALK mutation. EMBO J. (2021) doi:10.15252/embj.2020105784.
17. Sano, R. et al. An antibody-drug conjugate directed to the ALK receptor demonstrates efficacy in preclinical models of neuroblastoma. Sci. Transl. Med. 11, (2019).
18. Ishihara, T. et al. HEN-1, a secretory protein with an LDL receptor motif, regulates sensory integration and learning in Caenorhabditis elegans. Cell 109, 639-649 (2002).
19. Englund, C. et al. Jeb signals through the Alk receptor tyrosine kinase to drive visceral muscle fusion. Nature 425, 512-516 (2003).
20. Lee, H. H., Norris, A., Weiss, J. B. & Frasch, M. Jelly belly protein activates the receptor tyrosine kinase Alk to specify visceral muscle pioneers. Nature 425, 507-512 (2003).
21. Dornburg, A. et al. Comparative Genomics within and across Bilaterians Illuminates the Evolutionary History of ALK and LTK Proto-Oncogene Origination and Diversification. Genome Biol. Evol. 13, (2021).
22. US20200157236A1 - Anti-ALK Antibodies and Methods for Use Thereof - Google Patents.
23. Chothia, C., Gough, J., Vogel, C. & Teichmann, S. A. Evolution of the protein repertoire. Science vol. 300 1701-1703 (2003).
24. Crick, F. H. C. & Rich, A. Structure of polyglycine II. Nature 176, 780-781 (1955).
25. Pentelute, B. L. et al. X-ray structure of snow flea antifreeze protein determined by racemic crystallization of synthetic protein enantiomers. J. Am. Chem. Soc. 130, 9695-9701 (2008).
26. Buglino, J., Shen, V., Hakimian, P. & Lima, C. D. Structural and biochemical analysis of the Obg GTP binding protein. Structure 10, 1581-1592 (2002).
27. Weidenweber, S. et al. Structure of the acetophenone carboxylase core complex: Prototype of a new class of ATP-dependent carboxylases/hydrolases. Sci. Rep. 7, 1-10 (2017).
28. Dunne, M. et al. Salmonella Phage S16 Tail Fiber Adhesin Features a Rare Polyglycine Rich Domain for Host Recognition. Structure 26, 1573-1582.e4 (2018).
29. Lorén, C. E. et al. A crucial role for the Anaplastic lymphoma kinase receptor tyrosine kinase in gut development in Drosophila melanogaster. EMBO Rep. 4, 781-786 (2003).
30. Holm, L. DALI and the persistence of protein shape. Protein Sci. 29, 128-140 (2020).
31. Krupovic, M. & Koonin, E. V. Multiple origins of viral capsid proteins from cellular ancestors. Proc. Natl. Acad. Sci. U. S. A. 114, E2401-E2410 (2017).
32. Krissinel, E. & Henrick, K. Secondary-structure matching (SSM), a new tool for fast protein structure alignment in three dimensions. Acta Crystallogr. Sect. D Biol. Crystallogr. 60, 2256-2268 (2004).
33. Ressl, S. et al. Structures of C1q-like proteins reveal unique features among the C1q/TNF superfamily. Structure 23, 688-699 (2015).
34. Kolodny, R. Searching protein space for ancient sub-domain segments. Current Opinion in Structural Biology vol. 68 105-112 (2021).
35. Parmeggiani, F. & Huang, P. S. Designing repeat proteins: a modular approach to protein design. Current Opinion in Structural Biology vol. 45 116-123 (2017).
36. Lemmon, M. A. & Schlessinger, J. Cell signaling by receptor tyrosine kinases. Cell vol. 141 1117-1134 (2010).
37. Verstraete, K. & Savvides, S. N. Extracellular assembly and activation principles of oncogenic class III receptor tyrosine kinases. Nature Reviews Cancer vol. 12 753-766 (2012).
38. Verstraete, K. et al. Structural basis of the proinflammatory signaling complex mediated by TSLP. Nat. Struct. Mol. Biol. 21, 375-382 (2014).
39. Felix, J. et al. Structure and Assembly Mechanism of the Signaling Complex Mediated by Human CSF-1. Structure 23, 1621-1631 (2015).
40. Syed, R. S. et al. Efficiency of signalling through cytokine receptors depends critically on receptor orientation. Nature 395, 511-516 (1998).
41. De Vos, A. M., Ultsch, M. & Kossiakoff, A. A. Human growth hormone and extracellular domain of its receptor: Crystal structure of the complex. Science (80-. ). 255, 306-312 (1992).
42. Bloch, Y. et al. Structural Activation of Pro-inflammatory Human Cytokine IL-23 by Cognate IL-23 Receptor Enables Recruitment of the Shared Receptor IL-12Rβ1. Immunity 48, 45-58.e6 (2018).
43. Fadeev, A. et al. ALKALs are in vivo ligands for ALK family receptor tyrosine kinases in the neural crest and derived cells. Proc. Natl. Acad. Sci. U. S. A. 115, E630-E638 (2018).
44. Tate, J. G. et al. COSMIC: The Catalogue Of Somatic Mutations In Cancer. Nucleic Acids Res. 47, D941-D947 (2019).
45. Karczewski, K. J. et al. The mutational constraint spectrum quantified from variation in 141,456 humans. Nature 581, 434-443 (2020).
46. Wang, Y. W. et al. Identification of oncogenic point mutations and hyperphosphorylation of anaplastic lymphoma kinase in lung cancer. Neoplasia 13, 704-715 (2011).
47. Maxson, J. E. et al. Therapeutically targetable ALK mutations in leukemia. Cancer Res. 75, 2146-2150 (2015).
48. Durham, B. H. et al. Activating mutations in CSF1R and additional receptor tyrosine kinases in histiocytic neoplasms. Nat. Med. 25, (2019).
49. Malinauskas, T., Aricescu, A. R., Lu, W., Siebold, C. & Jones, E. Y. Modular mechanism of Wnt signaling inhibition by Wnt inhibitory factor 1. Nat. Struct. Mol. Biol. 18, 886-893 (2011).
50. Yang, H.-L. et al. The ligand Jelly Belly (Jeb) activates the Drosophila Alk RTK to drive PC12 cell differentiation, but is unable to activate the Mouse ALK RTK. J. Exp. Zool. Part B Mol. Dev. Evol. 308B, 269-282 (2007).
51. Wehrman, T. et al. Structural and Mechanistic Insights into Nerve Growth Factor Interactions with the TrkA and p75 Receptors. Neuron 53, 25-38 (2007).
52. Ogiso, H. et al. Crystal structure of the complex of human epidermal growth factor and receptor extracellular domains. Cell 110, 775-787 (2002).
53. Elegheert, J. et al. Extracellular complexes of the hematopoietic human and mouse CSF-1 receptor are driven by common assembly principles. Structure 19, 1762-1772 (2011).
54. Felix, J. et al. Human IL-34 and CSF-1 establish structurally similar extracellular assemblies with their common hematopoietic receptor. Structure 21, 528-539 (2013).
55. Schlessinger, J. et al. Crystal structure of a ternary FGF-FGFR-heparin complex reveals a dual role for heparin in FGFR binding and dimerization. Mol. Cell 6, 743-750 (2000).
56. Li, J. et al. Cryo-EM analyses reveal the common mechanism and diversification in the activation of RET by different ligands. Elife 8, (2019).
57. Chen, G. et al. α-Klotho is a non-enzymatic molecular scaffold for FGF23 hormone signalling. Nature 553, 461-466 (2018).
58. Uchikawa, E., Choi, E., Shang, G., Yu, H. & Xiao-Chen, B. Activation mechanism of the insulin receptor revealed by cryo-EM structure of the fully liganded receptor-ligand complex. Elife 8, (2019).
59. Moog-Lutz, C. et al. Activation and inhibition of anaplastic lymphoma kinase receptor tyrosine kinase by monoclonal antibodies and absence of agonist activity of pleiotrophin. J. Biol. Chem. 280, 26039-26048 (2005).
60. Aricescu, A. R., Lu, W. & Jones, E. Y. A time- and cost-efficient system for high-level protein production in mammalian cells. Acta Crystallogr. Sect. D Biol. Crystallogr. 62, 1243-1250 (2006).
61. Reeves, P. J., Callewaert, N., Contreras, R. & Khorana, H. G. Structure and function in rhodopsin: High-level expression of rhodopsin with restricted and homogeneous N-glycosylation by a tetracycline-inducible N-acetylglucosaminyltransferase I-negative HEK293S stable mammalian cell line. Proc. Natl. Acad. Sci. U. S. A. 99, 13419-13424 (2002).
62. Backliwal, G. et al. Valproic acid: A viable alternative to sodium butyrate for enhancing protein expression in mammalian cell cultures. Biotechnol. Bioeng. 101, 182-189 (2008).
63. Gorrec, F. The MORPHEUS II protein crystallization screen. Acta Crystallogr. Sect. Struct. Biol. Commun. 71, 831-837 (2015).
64. Kabsch, W. XDS. Acta Crystallogr. D. Biol. Crystallogr. 66, 125-32 (2010).
65. McCoy, A. J. et al. Phaser crystallographic software. J. Appl. Crystallogr. 40, 658-674 (2007).
66. Bricogne G., Blanc E., Brandl M., Flensburg C., Keller P., P. W. & Roversi P, Sharff A., Smart O.S., Vonrhein C., W. T. O. (2017). BUSTER 2.11.2. Cambridge, United Kingdom Glob. Phasing Ltd*.*
67. Emsley, P., Lohkamp, B., Scott, W. G. & Cowtan, K. Features and development of Coot. Acta Crystallogr. Sect. D Biol. Crystallogr. 66, 486-501 (2010).
68. Terwilliger, T. C. Maximum-likelihood density modification. Acta Crystallogr. Sect. D Biol. Crystallogr. 56, 965-972 (2000).
69. Liebschner, D. et al. Macromolecular structure determination using X-rays, neutrons and electrons: Recent developments in Phenix. Acta Crystallogr. Sect. D Struct. Biol. 75, 861-877 (2019).
70. Langer, G., Cohen, S. X., Lamzin, V. S. & Perrakis, A. Automated macromolecular model building for X-ray crystallography using ARP/wARP version 7. Nat. Protoc. 3, 1171-1179 (2008).
71. Murshudov, G. N. et al. REFMAC5 for the refinement of macromolecular crystal structures. Acta Crystallogr. Sect. D Biol. Crystallogr. 67, 355-367 (2011).
72. D'Arcy, A., Villard, F. & Marsh, M. An automated microseed matrix-screening method for protein crystallization. Acta Crystallogr. Sect. D Biol. Crystallogr. 63, 550-554 (2007).
73. Strong, M. et al. Toward the structural genomics of complexes: Crystal structure of a PE/PPE protein complex from Mycobacterium tuberculosis. Proc. Natl. Acad. Sci. U. S. A. 103, 8060-8065 (2006).
74. Howarth, M. & Ting, A. Y. Imaging proteins in live mammalian cells with biotin ligase and monovalent streptavidin. Nat. Protoc. 3, 534-545 (2008).
75. Hopkins, J. B., Gillilan, R. E. & Skou, S. BioXTAS RAW: Improvements to a free open-source program for small-angle X-ray scattering data reduction and analysis. J. Appl. Crystallogr. 50, 1545-1553 (2017).
76. Biasini, M. et al. SWISS-MODEL: Modelling protein tertiary and quaternary structure using evolutionary information. Nucleic Acids Res. 42, W252 (2014).
77. Webb, B. & Sali, A. Comparative protein structure modeling using MODELLER. Curr. Protoc. Bioinforma. 2016, 5.6.1-5.6.37 (2016).
78. Yoshimi, A. et al. Coordinated alterations in RNA splicing and epigenetic regulation drive leukaemogenesis. Nature 574, 273-277 (2019).

## Claims

1. Compositions in crystalline form selected from i)ALK_{TG}, depicted in SEQ ID NO: 1, interacting with ALKAL2, depicted in SEQ ID NO: 2, and ii) LTK_{TG}, depicted in SEQ ID NO: 3, interacting with ALKAL1, depicted in SEQ ID NO: 4, and Nb3.16, depicted in SEQ ID NO: 5, **characterized in that** the crystals are:
i) a crystal between ALK_{TG}, depicted in SEQ ID NO: 1, interacting with ALKAL2, depicted in SEQ ID NO: 2, in the space group P43212, with the following crystal lattice constants: a=97.57 Å ± 5%, b=97.57 Å ± 5%, c=355.35 Å ± 5%, α=β=γ=90°, and
ii) a crystal between LTK_{TG}, depicted in SEQ ID NO: 3, interacting with ALKAL1, depicted in SEQ ID NO: 4, and Nb3.16, depicted in SEQ ID NO: 5, in the space group P61, with the following crystal lattice constants: a=129.11 Å ± 5%, b=129.11 Å ± 5%, c=109.75 Å ± 5%, α=90°, β=90°, γ=120°.

2. The compositions of claim 1 which have a three-dimensional structure wherein the crystal i) comprises an atomic structure **characterized by** the coordinates depicted in the entry ID 7NWZ present in the on line RCSB protein database and wherein the crystal ii) comprises an atomic structure **characterized by** the coordinates depicted in the entry ID 7NX0 present in the on line RCSB protein database.

3. A computer-assisted method of identifying, designing or screening for a compound that can potentially interact with a crystal selected from a crystal i) or ii) as defined in claims 1 or 2, comprising performing structure-based identification, design or screening of a compound based on the compound's interactions with a structure defined by the atomic coordinates as defined in claim 2.

4. A method for identifying a compound that can bind to the complex i) ALK_{TG}, depicted in SEQ ID NO: 1, - ALKAL2, depicted in SEQ ID NO: 2, or to the complex ii) LTK_{TG}, depicted in SEQ ID NO: 3, - ALKAL1,depicted in SEQ ID NO: 4,- Nb3.16, depicted in SEQ ID NO: 5, comprising dipping candidate small molecule compounds with the complex ALK_{TG}, depicted in SEQ ID NO: 1, - ALKAL2,depicted in SEQ ID NO: 2, or the complex LTK_{TG}, depicted in SEQ ID NO: 3, - ALKAL1,depicted in SEQ ID NO: 4, - Nb3.16, depicted in SEQ ID NO: 5, and allowing co-crystallization, and screening candidate agonists or antagonists by using a method for measuring intermolecular interaction and comparing, designing and docking the 3D structures i) or ii) as defined in claim 2 and a candidate ligand by computer modeling.

5. A method for identifying an agonist or antagonist compound for the interaction with the complex i) ALK_{TG} ,depicted in SEQ ID NO: 1, - ALKAL2, depicted in SEQ ID NO: 2, or with the complex ii) LTK_{TG} , depicted in SEQ ID NO: 3, - ALKAL1, depicted in SEQ ID NO: 4, - Nb3.16, depicted in SEQ ID NO: 5, comprising an entity selected from the group consisting of an antibody, a peptide, a non-peptide molecule and a chemical compound, wherein said compound is capable of enhancing or disrupting the interaction of the complex i) ALK_{TG}, depicted in SEQ ID NO: 1, - ALKAL2, depicted in SEQ ID NO: 2, or the interaction of the complex ii) LTK_{TG}, depicted in SEQ ID NO: 3, - ALKAL1, depicted in SEQ ID NO: 4, - Nb3.16, depicted in SEQ ID NO: 5, wherein said process includes:
i) introducing into suitable computer program parameters defining an interacting surface based on the conformation of the complex i) ALK_{TG}, depicted in SEQ ID NO: 1, - ALKAL2 , depicted in SEQ ID NO: 2, or the complex ii) LTK_{TG}, depicted in SEQ ID NO: 3, - ALKAL1,depicted in SEQ ID NO: 4, - Nb3.16, depicted in SEQ ID NO: 5, corresponding to the atomic coordinates 7NWZ or 7NX0 present in the on line RCSB protein database, wherein said program displays a three-dimensional model of the interacting surface,
ii) creating a three-dimensional structure of a test compound in said computer program;
iii) displaying a superimposing model of said test compound on the three-dimensional model of the interacting surface;
iv) and assessing whether said test compound model fits spatially into an interaction site.

## Patentansprüche

1. Zusammensetzungen in kristalliner Form, ausgewählt aus i) ALK_{TG}, in SEQ ID Nr. 1 dargestellt, die mit ALKAL2, in SEQ ID Nr. 2 dargestellt, interagiert, und ii) LTK_{TG}, in SEQ ID Nr. 3 dargestellt, die mit ALKAL1, in SEQ ID Nr. 4 dargestellt, und Nb3.16, in SEQ ID Nr. 5 dargestellt, interagiert, **dadurch gekennzeichnet, dass** die Kristalle sind:
i) ein Kristall zwischen ALK_{TG}, in SEQ ID Nr. 1 dargestellt, die mit ALKAL2, in SEQ ID Nr. 2 dargestellt, interagiert, in der Raumgruppe P43212, mit den folgenden Kristallgitterkonstanten: a = 97,57 Å ± 5 %, b = 97,57 Å ± 5 %, c = 355,35 Å ± 5 %, α = β = γ = 90°, und
ii) ein Kristall zwischen LTK_{TG}, in SEQ ID Nr. 3 dargestellt, die mit ALKAL1, in SEQ ID Nr. 4 dargestellt, und Nb3.16, in SEQ ID Nr. 5 dargestellt, interagiert, in der Raumgruppe P61, mit den folgenden Kristallgitterkonstanten: a = 129,11 Å ± 5 %, b = 129,11 Å ± 5 %, c = 109,75 Å ± 5 %, α = 90°, β = 90°, γ = 120°.

2. Zusammensetzungen nach Anspruch 1, die eine dreidimensionale Struktur aufweisen, wobei der Kristall i) eine Atomstruktur umfasst, die durch die Koordinaten gekennzeichnet ist, die in der Eintrags-ID 7NWZ dargestellt sind, die in der Online-RCSB-Proteindatenbank vorliegt, und wobei der Kristall ii) eine Atomstruktur umfasst, die durch die Koordinaten gekennzeichnet ist, die in der Eintrags-ID 7NX0 dargestellt sind, die in der Online-RCSB-Proteindatenbank vorliegt.

3. Computergestütztes Verfahren zum Identifizieren, Entwerfen oder Screenen auf eine Verbindung, die potenziell mit einem Kristall interagieren kann, der aus einem Kristall i) oder ii) wie in den Ansprüchen 1 oder 2 definiert ausgewählt ist, umfassend ein Durchführen einer strukturbasierten Identifikation, eines strukturbasierten Entwurfs oder Screenens einer Verbindung auf der Basis der Interaktionen der Verbindung mit einer Struktur, die durch die Atomkoordinaten wie in Anspruch 2 definiert ist.

4. Verfahren zum Identifizieren einer Verbindung, die an den Komplex i) ALK_{TG}, in SEQ ID Nr. 1 dargestellt, - ALKAL2, in SEQ ID Nr. 2 dargestellt, oder an den Komplex ii) LTK_{TG}, in SEQ ID Nr. 3 dargestellt, - ALKAL1, in SEQ ID Nr. 4 dargestellt, - Nb3.16, in SEQ ID Nr. 5 dargestellt, binden kann, umfassend ein Dippen von Kleinmolekül-Kandidatenverbindungen mit dem Komplex ALK_{TG}, in SEQ ID Nr. 1 dargestellt, - ALKAL2, in SEQ ID Nr. 2 dargestellt, oder dem Komplex LTK_{TG}, in SEQ ID Nr. 3 dargestellt, - ALKAL1, in SEQ ID Nr. 4 dargestellt, - Nb3.16, in SEQ ID Nr. 5 dargestellt, und ein Zulassen einer Kokristallisation und ein Screenen von Kandidatenagonisten oder -antagonisten durch Verwenden eines Verfahrens zum Messen von intermolekularer Interaktion und Vergleichen, Entwerfen und Docken der 3D-Strukturen i) oder ii) wie in Anspruch 2 definiert und eines Kandidatenliganden durch Computermodellierung.

5. Verfahren zum Identifizieren einer Agonisten- oder Antagonistenverbindung zur Interaktion mit dem Komplex i) ALK_{TG}, in SEQ ID Nr. 1 dargestellt, - ALKAL2, in SEQ ID Nr. 2 dargestellt, oder mit dem Komplex ii) LTK_{TG}, in SEQ ID Nr. 3 dargestellt, - ALKAL1, in SEQ ID Nr. 4 dargestellt, - Nb3.16, in SEQ ID Nr. 5 dargestellt, umfassend eine Entität, die aus der Gruppe bestehend aus einem Antikörper, einem Peptid, einem Nicht-Peptid-Molekül und einer chemischen Verbindung ausgewählt ist, wobei die Verbindung dazu befähigt ist, die Interaktion des Komplexes i) ALK_{TG}, in SEQ ID Nr. 1 dargestellt, - ALKAL2, in SEQ ID Nr. 2 dargestellt, oder die Interaktion des Komplexes ii) LTK_{TG}, in SEQ ID Nr. 3 dargestellt, - ALKAL1, in SEQ ID Nr. 4 dargestellt, - Nb3.16, in SEQ ID Nr. 5 dargestellt, zu verstärken oder zu stören, wobei das Verfahren einschließt:
i) Eintragen von Parametern in ein geeignetes Computerprogramm, die eine interagierende Oberfläche auf der Basis der Konformation des Komplexes i) ALK_{TG}, in SEQ ID Nr. 1 dargestellt, - ALKAL2, in SEQ ID Nr. 2 dargestellt, oder des Komplexes ii) LTK_{TG}, in SEQ ID Nr. 3 dargestellt, - ALKAL1, in SEQ ID Nr. 4 dargestellt, - Nb3.16, in SEQ ID Nr. 5 dargestellt, definieren, entsprechend den Atomkoordinaten 7NWZ oder 7NX0, die in der Online-RCSB-Proteindatenbank vorliegen, wobei das Programm ein dreidimensionales Modell der interagierenden Oberfläche anzeigt,
ii) Erzeugen einer dreidimensionalen Struktur einer Testverbindung in dem Computerprogramm;
iii) Anzeigen eines überlagernden Modells der Testverbindung auf dem dreidimensionalen Modell der interagierenden Oberfläche;
iv) und Beurteilen, ob das Testverbindungsmodell räumlich in eine Interaktionsstelle passt.

## Revendications

1. Compositions sous forme cristalline sélectionnées parmi i) ALK_{TG}, représentée dans SEQ ID N° : 1, interagissant avec ALKAL2, représentée dans SEQ ID N° : 2, et ii) LTK_{TG}, représentée dans SEQ ID N° : 3, interagissant avec ALKAL1, représentée dans SEQ ID N° : 4, et Nb3.16, représentée dans SEQ ID N° : 5, **caractérisées en ce que** les cristaux sont :
i) un cristal entre ALK_{TG}, représentée dans SEQ ID N° : 1, interagissant avec ALKAL2, représentée dans SEQ ID N° : 2, dans le groupe spatial P43212, avec les constantes de réseau cristallin suivantes : a=97.57 Å ± 5 %, b=97.57 Å ± 5 %, c=355.35 Å ± 5 %, α=β=γ=90°, et
ii) un cristal entre LTK_{TG}, représentée dans SEQ ID N° : 3, interagissant avec ALKAL1, représentée dans SEQ ID N° : 4, et Nb3.16, représentée dans SEQ ID N° : 5, dans le groupe spatial P61, avec les constantes de réseau cristallin suivantes : a=129.11 Å ± 5 %, b=129.11 Å ± 5 %, c=109.75 Å ± 5 %, α=90°, β=90°, γ=120°.

2. Compositions selon la revendication 1 qui ont une structure tridimensionnelle, dans lesquelles le cristal i) comprend une structure atomique **caractérisée par** les coordonnées représentées dans l'identifiant d'entrée 7NWZ présent dans la base de données RCSB en ligne sur les protéines, et dans lesquelles le cristal ii) comprend une structure atomique **caractérisée par** les coordonnées représentées dans l'identifiant d'entrée 7NX0 présent dans la base de données RCSB en ligne sur les protéines.

3. Procédé assisté par ordinateur d'identification, de conception ou de criblage pour un composé qui peut potentiellement interagir avec un cristal sélectionné parmi un cristal i) ou ii) tel que défini dans la revendication 1 ou 2, comprenant réaliser une identification, une conception ou un criblage basé(e) sur la structure d'un composé en fonction d'interactions du composé avec une structure définie par les coordonnées atomiques telle que définie dans la revendication 2.

4. Procédé d'identification d'un composé qui peut se lier au complexe i) ALK_{TG}, représenté dans SEQ ID N° : 1, - ALKAL2, représenté dans SEQ ID N° 2, ou au complexe ii) LTK_{TG}, représenté dans SEQ ID N° : 3, - ALKAL1, représenté dans SEQ ID N° : 4,- Nb3.16, représenté dans SEQ ID N° : 5, comprenant tremper des composés à petite molécule candidats avec le complexe ALK_{TG}, représenté dans SEQ ID N° : 1, - ALKAL2, représenté dans SEQ ID N° : 2, ou le complexe LTK_{TG}, représenté dans SEQ ID N° : 3, - ALKAL1, représenté dans SEQ ID N° : 4, - Nb3.16, représenté dans SEQ ID N° : 5, et permettre une co-cristallisation, et cribler des agonistes ou antagonistes candidats en utilisant un procédé de mesure d'interaction intermoléculaire, et comparer, concevoir et amarrer les structures tridimensionnelles i) ou ii) telles que définies dans la revendication 2 et un ligand candidat par modélisation informatique.

5. Procédé d'identification d'un composé agoniste ou antagoniste pour l'interaction avec le complexe i) ALK_{TG} , représenté dans SEQ ID N° : 1, - ALKAL2, représenté dans SEQ ID N° : 2, ou avec le complexe ii) LTK_{TG} , représenté dans SEQ ID N° : 3, - ALKAL1, représenté dans SEQ ID N° : 4, - Nb3.16, représenté dans SEQ ID N° : 5, comprenant une entité sélectionnée parmi le groupe constitué d'un anticorps, d'un peptide, d'une molécule non peptidique et d'un composé chimique, dans lequel ledit composé est capable d'amplifier ou de perturber l'interaction du complexe i) ALK_{TG}, représenté dans SEQ ID N° : 1, - ALKAL2, représenté dans SEQ ID N° : 2, ou l'interaction du complexe ii) LTK_{TG}, représenté dans SEQ ID N° : 3, - ALKAL1, représenté dans SEQ ID N° : 4, - Nb3.16, représenté dans SEQ ID N° : 5, dans lequel ledit processus inclut :
i) introduire dans un programme informatique approprié des paramètres définissant une surface d'interaction basée sur la conformation du complexe i) ALK_{TG}, représenté dans SEQ ID N° : 1, - ALKAL2 , représenté dans SEQ ID N° : 2, ou du complexe ii) LTK_{TG}, représenté dans SEQ ID N° : 3, - ALKAL1, représenté dans SEQ ID N° : 4, - Nb3.16, représenté dans SEQ ID N° : 5, correspondant aux coordonnées atomiques 7NWZ ou 7NX0 présentes dans la base de données RCSB en ligne sur les protéines, dans lequel ledit programme affiche un modèle tridimensionnel de la surface d'interaction,
ii) créer une structure tridimensionnelle d'un composé d'essai dans ledit programme informatique ;
iii) afficher un modèle de superposition dudit composé d'essai sur le modèle tridimensionnel de la surface d'interaction ;
iv) et évaluer si ledit composé d'essai s'ajuste spatialement dans un site d'interaction.
